# EUROPEAN PATENT SPECIFICATION

(11) **EP 3 420 071 B1**
(45) Date of publication and mention of the grant of the patent: **15.07.2020**
(21) Application number: 17706979.6
(22) Date of filing: 21.02.2017
(51) Int. Cl.: C12N 5/00, C12M 1/12, C12Q 1/02

(54) **A NEURONAL CELL CULTURE SUBSTRATE AND IN VITRO METHODS OF USING THEREOF**
NEURONALES ZELLKULTURSUBSTRAT UND IN VITRO-VERFAHREN ZUR VERWENDUNG DAVON
SUBSTRAT DE CULTURE DE CELLULES NEURONALES ET PROCÉDÉ IN VITRO UTILISANT LEDIT SUBSTRAT

(30) Priority: 25.02.2016 IT UB20161057
(43) Date of publication of application: 02.01.2019
(73) Proprietor: Fondazione Istituto Italiano di Tecnologia, 16163 Genova (IT)
(72) Inventor: AMIN, Hayder, 16010 Manesseno (Genova) (IT); BERDONDINI, Luca, 16128 Genova (IT)
(74) Representative: Comoglio, Elena
(86) International application number: PCT/EP2017/053911
(87) International publication number: WO 2017/144452

(56) References cited:
- RICHARDSON G P ET AL: "Torpedo electromotor system development: Developmentally regulated neuronotrophic activities of electric organ tissue", JOURNAL OF COMPARATIVE NEUROLOGY, vol. 231, no. 3, 15 January 1985 (1985-01-15), pages 339-352, XP055319471, US ISSN: 0021-9967, DOI: 10.1002/cne.902310305
- LIANG S & CRUTCHER K A: "Neuronal migration on laminin in vitro", DEVELOPMENTAL BRAIN RESEARCH, vol. 66, no. 1, 20 March 1992 (1992-03-20) , pages 127-132, XP024334702, NL ISSN: 0165-3806, DOI: 10.1016/0165-3806(92)90148-P [retrieved on 1992-03-20]
- NOELL S ET AL: "Effects of agrin on the expression and distribution of the water channel protein aquaporin-4 and volume regulation in cultured astrocytes", EUROPEAN JOURNAL OF NEUROSCIENCE, vol. 26, no. 8, 10 October 2007 (2007-10-10), pages 2109-2118, XP055319463, GB ISSN: 0953-816X, DOI: 10.1111/j.1460-9568.2007.05850.x
- ZUÑIGA-AGUILAR E ET AL: "Nerve cells culture from lumbar spinal cord on surfaces modified by plasma pyrrole polymerization", JOURNAL OF BIOMATERIALS SCIENCE, POLYMER EDITION, vol. 25, no. 7, 3 May 2014 (2014-05-03), pages 729-747, XP055319651, NL ISSN: 0920-5063, DOI: 10.1080/09205063.2014.898124
- AMIN H ET AL: "Electrical Responses and Spontaneous Activity of Human iPS-Derived Neuronal Networks Characterized for 3-month Culture with 4096-Electrode Arrays", FRONTIERS IN NEUROSCIENCE, vol. 10, 121, 30 March 2016 (2016-03-30), XP055319452, ISSN: 1662-4548, DOI: 10.1016/S0896-6273(00)80409-2

## Description

The present invention relates to a neuronal cell culture substrate and to an *in vitro* method of promoting adhesion, survival and/or proliferation of neuronal cells in culture as well as to an *in vitro* method of promoting neuronal network growth and/or maturation.

The cultivation of living cells *in vitro* by means of cell-culture methods is a major tool in neuroscience and biomedical research. Simplified *in vitro* neuronal networks may reminisce the neural cell behaviors *in vivo,* i.e. proliferation, differentiation, and migration, and offer a promising approach to study existing neuronal models and to gain profound understanding of their related biological processes throughout potential applications, such as the development of cell-based biosensors, and to assess the toxicological and/or neuroprotective effects of pharmaceutical compounds Thus, to generate an appropriate physiological environment for these neuronal cultures, cell adhesion and growth is pivotal and requires a special biochemical modification of the surface substrate.

Generally, for most cells, especially for post-mitotic neurons, coated surfaces are a prerequisite for seeding. Indeed, cells have a negatively charged surface that constituted by glycoproteins and glycolipids in their plasma membrane. Thus, a variety of biologically purified adhesive molecules such as collagen, laminin, and fibronectin, as well as basic synthetic polymers, such as poly-D-lysine (PDL), poly-L-lysine (PLL), and polyethylenimine (PEI) have been used to generate a positively charged layer, to enhance the cell attachment in serum-free and low serum conditions.

US 2009/0311735 discloses a method of promoting the attachment, survival and/or proliferation of a stem cell in culture, the method comprising culturing a stem cell on a positively-charged support surface. According to an embodiment disclosed in US 2009/0311735, the support surface may comprise a solid, preferably polymeric support to which a positively-charged molecule is bound. Trimethylammonium polyetherimide, poly-L-lysine, poly-D-lysine (PDL), poly-L-ornithine (PLO), poly-D-ornithine (PDO), poly(vinylamine) (PVA), poly(allylamine) (PAA)), or combinations thereof are mentioned as suitable positively-charged molecules.

Over the past several years, albeit basic cell-assays using polystyrene and glass substrates, the need has been emerged for developing neuro-electrode based devices for long-term noninvasive extracellular recordings that allow to record simultaneously from many neurons, and hence, to monitor their network functional changes over development and/or as responses to electrical stimuli, chemical manipulations and/or compounds. Thus, the success of such approach depends on a suitable bio-functionalization method to allow viable development of the neuronal network up to an age, where the neuronal networks establish their functional physiological properties.

While variety of surface treatments have been investigated for attachment of neurons on polystyrene, glass, silicon, and metal surfaces, no salient biochemical molecules has been suggested uniquely for mastering any type of surfaces and as being appropriate for different sources of neurons, such as from rat, mouse, and/or human.

Thus, there is a need for a unique biochemical molecule that may be suitable for coating any surface prior to cell seeding.

These and other needs are met by the present invention, which provides the neuronal cell culture substrate according to independent claim 1.

The invention also provides an *in vitro* method of promoting adhesion, survival and/or proliferation of neuronal cells in culture as defined in independent claims 9 and to an *in vitro* method of promoting neuronal network growth and/or maturation as defined in independent claim 10. Within the present description, the expression "adhesion" may optionally include electrode-neuron sealing for electrical recordings, since the inventors observed extremely high signal amplitudes when recording form neurons grown on PDLO-coated electrode arrays. The high amplitude results from high adhesion and sealing on the electrode surface.

Further features and advantages of the invention are as illustrated in the dependent claims. The claims form an integral part of the present patent description.

The use of the neuronal cell culture substrate comprising a neuronal cell culture support surface coated with poly-DL-ornithine (PDLO) according to the present invention provides for morphological and functional cellular enhancement of neuronal cultures regardless their source, such as mammalian, e.g. rat, mouse, or human. The growth of neurons on PDLO coated surfaces according to the invention shows drastically better physiological development in comparison with other standard coatings.

The inventors studied the PDLO effect on the growth, formation of functional network of human-derived neuronal networks grown on CMOS-multi-electrode arrays, glass coverslips and polystyrene surfaces for three months in serum-free and glia-free *in vitro* cultures. It has to be highlighted that these cells are particularly difficult to grow and none of the existing methods allows to achieve similar network properties as obtained with the proposed method. These cell, under conditions of serum-free and glia-free cell culture (i.e. a worst-case cell culture condition), are therefore an adequate model to demonstrate the proposed method. The same human-derived neuronal cell-line used by the present inventors, i.e. iCell neurons™ (International Cellular Dynamics, USA), has been reported in (Odawara, 2014), where authors characterized the neuronal development on-chip by using conventional multielectrode arrays recording from 60-electrodes. In Odawara 2014, neurons were grown on PEI substrates for up to three months in the present of conditional glial-medium and glial-feeder cultures. Odawara 2014 reported the disability of neuronal network grown on PEI substrates to show persistent electrical activity in glia-free cultures. Thus, the inventors remarkably found that glia-neuron co-cultures are not mandatory to achieve network growth and maturation; rather, an optimized surface functionalization utilizing PDLO is essential. The method of the invention can be combined with glial-neuron co-cultures or with the use of serum for specific studies.

The following Experimental Section is provided to further illustrate the invention and it is not intended to limit the scope of the invention, which is defined by the appended claims.

In the Experimental Section, the inventors investigated the morphological and functional effects induced by three different biochemically active molecules deposited on the chip surfaces before cellular seeding and evaluated their ability to support cellular attachment, culture viability over several months, and to enhance neuroelectronic coupling. In particular, commercially available iCell neuronal cultures were grown on the CMOS-MEA and on glass surfaces coated with PDLO, PEI, and PLO. The inventors quantified the growth of these cultures with the use of fluorescence and confocal microscopy for up to 90 DIV even though morphological differences with respect to other coating substrates had been observed already after 1 DIV. Morphologically, surfaces that were coated with PDLO and PEI showed homogeneous cellular growth and maintained distributed individual cells, while cultures grown on PLO coated substrates showed a strong prominence to the formation of cellular clusters and fasciculation of the neuritic processes. In addition, networks grown on PLO coated CMOS-MEAs did not show neither spontaneous activity nor evoked responses, despite the high-electrode density that is expected to allow for recordings also in the case of a very low number of spiking neurons. On the other side, spontaneous electrical activity was recorded from CMOS-MEAs coated with PDLO or PEI, with significant differences induced by the two coatings during development. Evoked responses to electrical stimulation were recorded only from PDLO-coated devices.

The experimental Section makes reference to the appended drawings, in which:

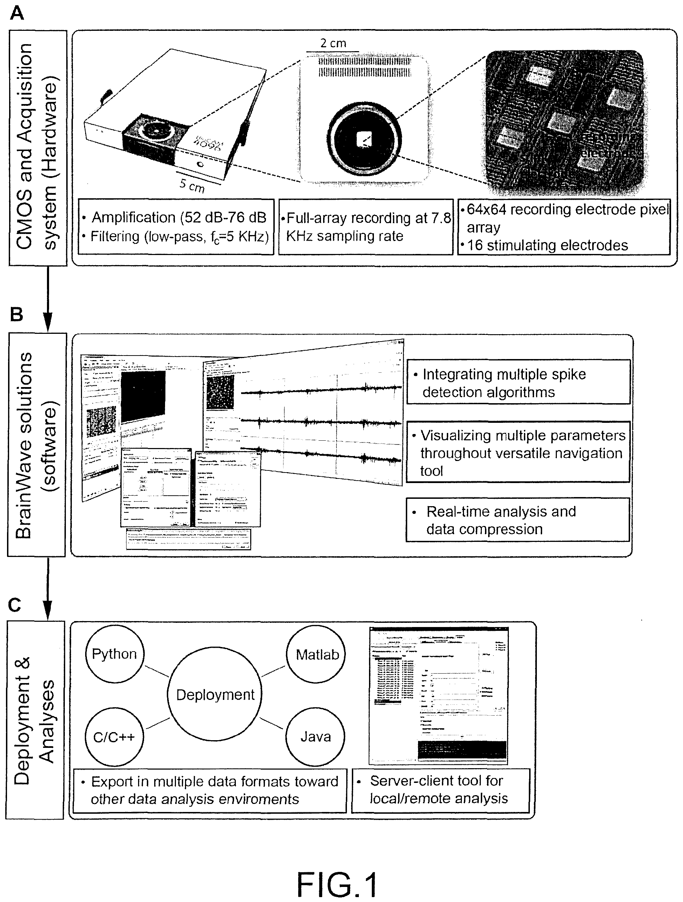

Figure 1 is an overview of the high-resolution electrophysiological platform based on CMOS multielectrode array (MEA) technology used in this work. (A) (from left to right); Real-time hardware for control and data acquisition, an image of a bi-directional CMOS-MEA that feature electrical recording and stimulation electrodes on-chip (3Brain GmbH, Switzerland), and a scanning electron microscope (SEM) image on an area of the electrode array. (B) Screenshot of the BrainWave software application that is used for managing the electrophysiological experiments (3Brain GmbH, Switzerland). (C) Overview of a custom software tool for data deployment and analysis, with a custom server-client tool (Maccione et al. 2015) for data analysis with minimized data transfer.

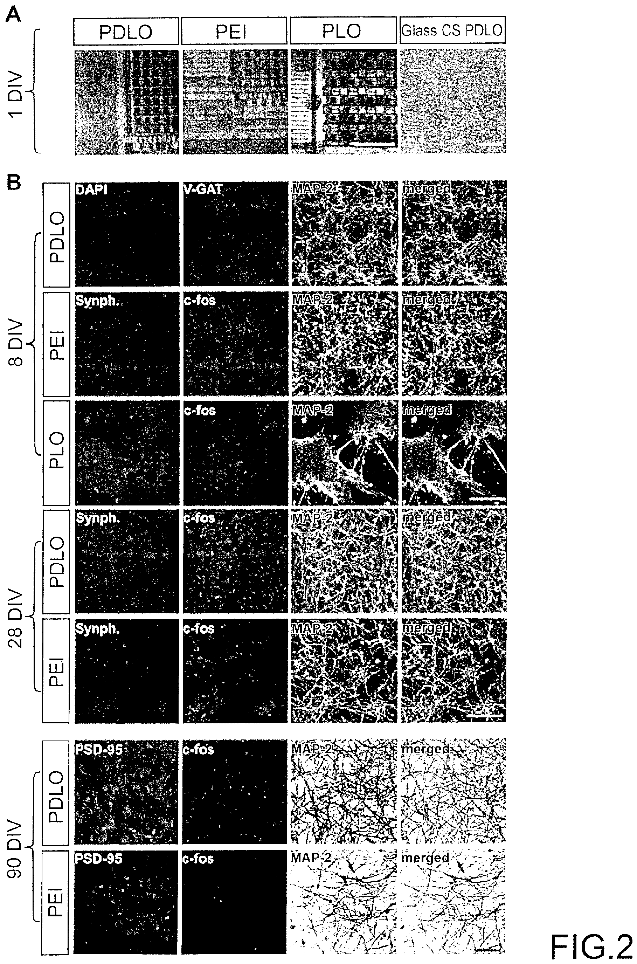

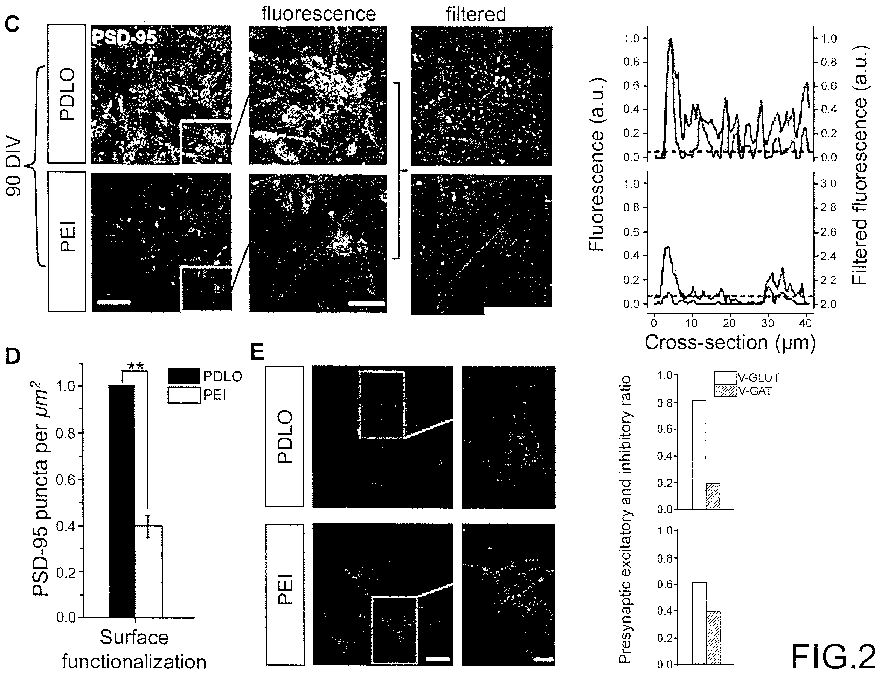

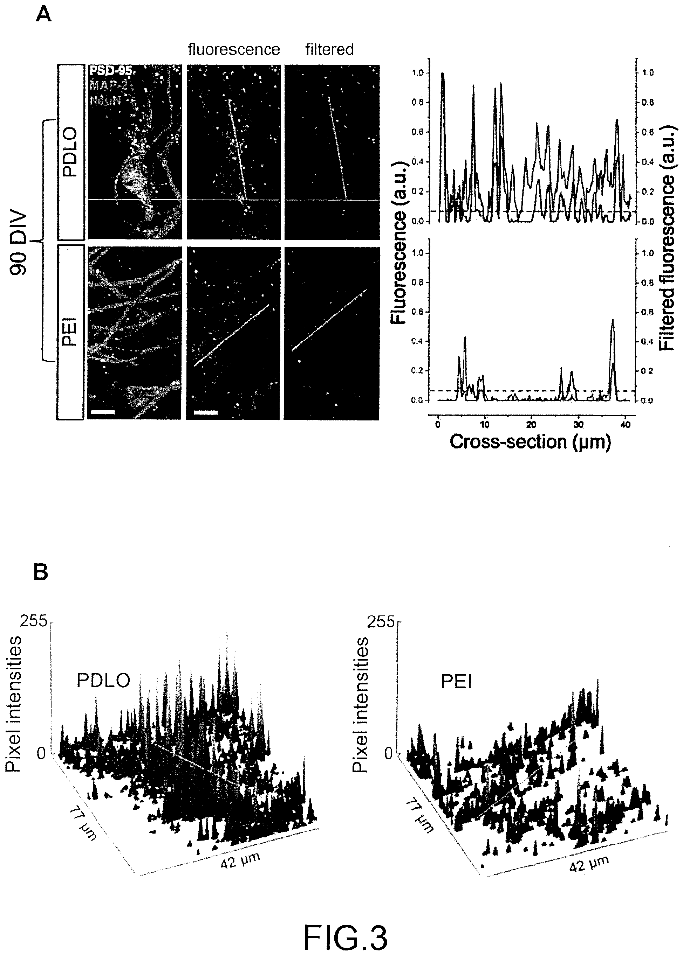

Figure 2 shows the optical read-outs that allow to track the development of human iCell neuronal networks over three months culture and the effect of biochemical adhesion molecules on neuronal growth and function. (A) Fluorescence images elucidate differences in neuronal growth after 1 DIV on CMOS-MEAs and glass coverslips coated with PDLO, PEI, and PLO adhesion molecules. Cultures grow homogenously on PDLO and PEI, but they cluster and fascicule on PLO surfaces. Scale bars represent 100 µm, and 50 µm, from left to right, respectively. (B) Confocal micrographs of iCell neurons™ at 8, 28, and 90 DIV show a heterogonous expression of typical neuronal and synaptic proteins, i.e. Synaptophysin-1, and GAD-65 (blue), V-GAT, and c-FOS (red), in addition to PSD-95 and MAP-2 (green). These immunostained images confirm that neurons grown on PDLO and PEI yielded healthy and homogeneous neurite formation and extension, whereas, neurons grown on PLO exhibit bundled-like neurite processes. In PLO cultures, despite they expressed cellular neuronal activity indicated by c-FOS, after 3 weeks, cultures adhesion has been lost, i.e. tissue lifted upward and thus cultures were wash-out. At 28 DIV, neurons grow on PDLO and PEI show no distinguishable differences between their cellular neuronal activity (c-FOS) and presynaptic expression (synaptophysin-1), whereas at 90 DIV, the expression of postsynaptic protein PSD-95 is prominently higher in cultures grown on PDLO than others grown on PEI, which provides evidence of different maturation profiles. Scale bars represent 100 µm for (8, and 28 DIV), and 50 µm for 90 DIV. (C) Cross-sections along the indicated cut regions (magnified) elucidate the intensity and density of PSD-95 constructs. To quantify the puncta of PSD-95 constructs, images were processed with the granulometric filtering (see Materials and Methods and Figure 3). Blue and red cross-sections depict the fluorescence and the corresponding filtered intensities, respectively. The position of PSD-95 puncta was defined above an arbitrary offset, i.e. 0.07, here indicated by yellow bars. Scale bars represent 50 µm (left), and 20 µm (middle and right) for the magnified region. (D) Bar graph showing the quantified effect of surface functionalization on the expression of PSD-95 ensembles (density). Neurons grown on PDLO show significantly higher PSD-95 level than those grown on PEI (n=3, at least 9 image fields per sample, **p < 0.01). (E) At 90 DIV, quantifications performed on confocal micrographs (n=3, at least 9 image fields per sample) illustrate lower expression of V-GAT in neurons grown on PDLO than those grown on PEI. Thus, the ratio of excitatory-to-inhibitory synapses indicated by the ratio of V-GLUT/V-GAT is higher in neuronal ensembles grown on PDLO coated substrates, while it is lower for neurons on PEI, suggesting an effect of the surface functionalization on the developing balance of excitatory-to-inhibitory synaptic input. Scale bars represent 50 µm (left), and 20 µm (right) for the magnified region.
Figure 3 shows the effect of biochemical adhesion molecules on the PSD-95 expression in low-density iCell neuronal cultures. (A) Confocal micrographs of low-dense neurons (40'000 cells per CMOS-MEA "BioChip 4096E"), grown on PDLO and PEI substrates are fixed and stained at 90 DIV for dendritic marker (MAP-2), neuronal nuclear marker (NeuN), and the postsynaptic density marker (PSD-95). Cross-sections along the indicated regions elucidate the intensity and density of PSD-95 constructs. To quantify the puncta of PSD-95 constructs, images were processed with the granulometric filtering (see Materials and Methods, and Figure 2C). Blue and red cross-sections depict the fluorescence and the corresponding filtered intensities, respectively, thus indicating a significant higher puncta construct of PSD-95 in neurons grown on PDLO, than lower puncta in those grown on PEI. The position of PSD-95 puncta was defined above an arbitrary offset, i.e. 0.07, here indicated by yellow bars. Scale bars represent 10 µm. (B) 3D surface plots of full areas of the indicated micrographs in (A) displaying the pixel intensities in pseudo color image. Red cross-sections illustrate in 3D, the position of the quantified intensity and density of PSD-95 in 2D images in (A).

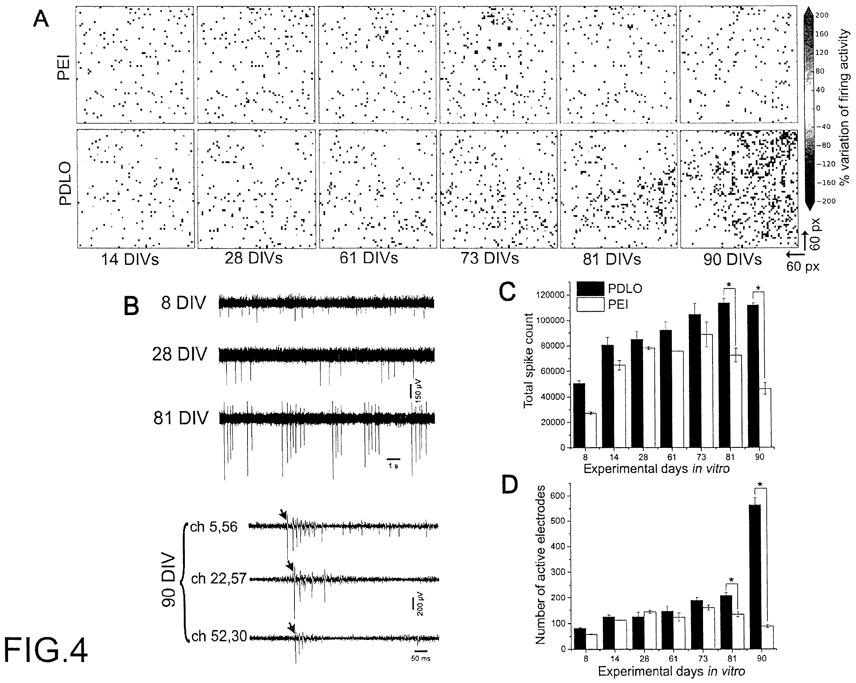

Figure 4 shows the high-resolution electrical read-outs with CMOS-MEAs, which unveil changes in spiking activity of iCell neuronal networks during development. (A) Activity maps obtained with the 4096 CMOS-MEA show the developmental changes in the spontaneous electrical activity of human-derived neurons over maturation (14, 28, 61, 73, 81, and 90 DIV) with respect to the first recorded time-set at 8 DIV. A prominent increase in the firing activity (as indicated by red pixels in the variation maps) is observed at 81 and at 90 DIV for neuronal networks grown on PDLO coated substrates, whilst the ones on PEI display a modest increase of the firing activity at 73 DIV, followed by a substantial decay. Color-code bar indicates the percentage of variation of firing rates for all recorded phases in respect to 8 DIV, where red is for increasing activity, and blue for decreasing activity. (B) Typically recorded extracellular signal traces associated with the detected firing rates during development. The activity develops from single spikes (8 DIV), tonic firing (28 DIV) to bursting and synchronized spikes (81, and 90 DIV). (C) Bar graph showing the effect of surface functionalization on the evolution of network-wide spiking activity indicated by the spike counts. Spontaneous firing rates of cultures grown on PDLO coated substrates tend to gradually increase during development to reach a peak at 81 DIV, followed by a plateau at 90 DIV. A similar, rather lower rate, trend in firing rates is observed for cultures grown on PEI coated substrates cultures follow the same increasing tendency of PDLO cultures. These firing rate values reach their peak at 73 DIV, earlier than PDLO cultures. Afterward, firing activity significantly declines at 81, and 90 DIV (*p < 0.05). (D) Bar graph illustrating the dynamic changes in the total number of active electrodes over three months of culture, for human-derived neurons grown on PDLO and PEI coated substrates. Consistently with the development of the spiking activity shown in (C), the number of active electrodes indicates an increasing tendency. A higher increase is observed for cultures grown on PDLO than PEI and this tendency becomes significantly higher for cultures on PDLO at 81, and 90 DIV (*p < 0.05).

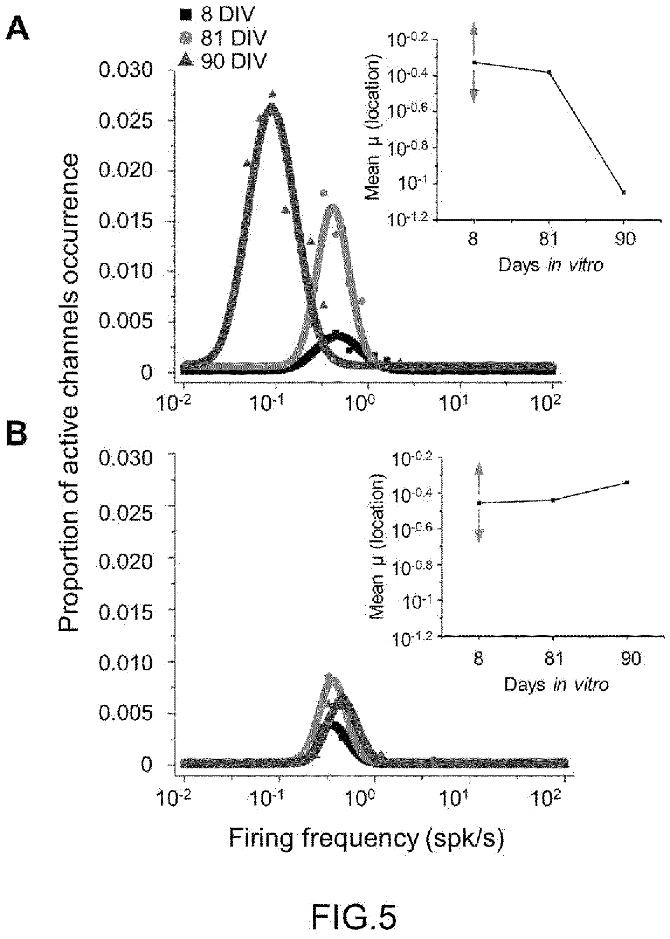

Figure 5 shows the distributions of the firing frequencies of iCell neuronal networks, which are lognormal- like and allow to elucidate the dynamic behavior of human iCell neuronal network during development. (A) The lognormal-like distribution represents the network dynamics of human derived neurons; Gaussian fits of the firing frequency distributions for three developmental phases of neurons grown on PDLO coated substrates, i.e. at 8, 81, 90 DIV. The means of the distributions at 8, and 81 DIV hold the same frequency position, but a higher peak of the distribution (red) is observed at 81 DIV. At 90 DIV, the mean of the distribution (blue) shifts left toward low-firing rates and the occurrence of active electrodes reaches its peak at this range of low frequency. Inset summarizes by use of the mean µ, the position of the Gaussian fit peak of each recording phase to evaluate the frequency shift from high toward low rates. Arrows on the y-axis show the shift directions on the logarithmic scale, where (↑), refers to the right, and (↓) refers to the left. (B) Similarly, lognormal-like distribution of neuronal cultures grown on PEI coated substrates and over cell culture. The mean of the distributions for all these phases hold the same frequency range, in addition, the peak of these distributions indicates a lower probability of active electrode occurrence during development. Inset displays the unchanged position of the Gaussian fit peak as indicated by the mean µ, for three recording phases during development, where no frequency shift occurred. Arrows on the y-axis show the shift direction on the logarithmic scale, where (↑), refers to the right, and (↓) refers to the left.

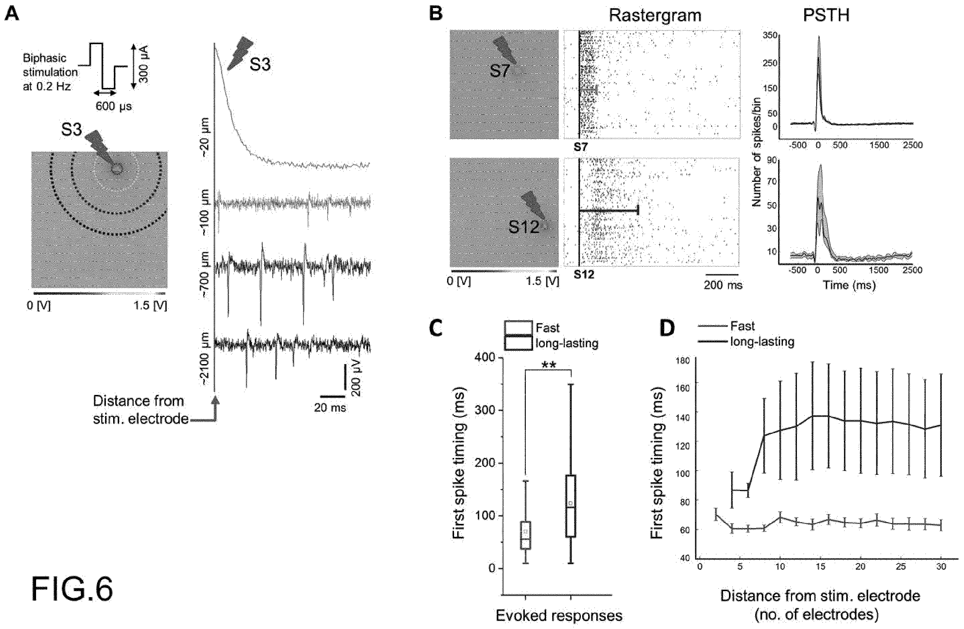

Figure 6 shows that electrical stimulation delivered by electrodes on-chip show evoked responses only at 90 DIV. (A) Sample of evoked signal traces illustrates the confined stimulation artifact to electrodes close to the stimulating electrodes (<100 µm), e.g. when low-frequency (0.2 Hz) trains of biphasic current stimuli (300 µs per phase, peak-to-peak amplitude of 300 µA) is delivered for 3 min to exemplary electrode S3. Colors of signal traces correspond to the distance from the stimulating site that is correlated with colors of dotted circles in the 64 x 64 array, e.g. green response occurred at ∼6 electrodes distance (∼100 µm) from the stimulating site, while blue at ∼15 electrode distance (∼700 µm), and black trace at ∼26 electrode distance (∼2100 µm). (B) Exemplary electrical stimuli (S7 and S12) delivered by specific electrodes on the 4096 electrode arrays (left) and their corresponding evoked responses by means of rastergram and PSTH analyses (middle and right, respectively). Two forms of evoked responses were observed and show, fast responses lasting < 100 ms (top), and long-lasting responses up to ∼500 ms (bottom). In the middle, rastergrams showing a sequence of evoked spikes that are confined within the green line (top), while evoked spikes are spread along the blue line (bottom). On the right, exemplary PSTHs that are computed from all evoked activities corresponding to their stimulating electrodes (see Materials and Methods). This shows that fast responses are characterized by a higher number of spikes per 25 ms bin size and lasting < 100 ms, in contrast with a lower number of spikes per bin and duration up to ∼500 ms in case of long-lasting responses. (C) Average of the first spike timing delays with respect to electrical stimulation, and collectively measured. A delay of about 70 ms is observed from fast responses (green), while the onset of long-lasting responses (blue) is observed over i.e. 120 ms. (n=3, **p < 0.01). (D) The average of first spike timing is measured with respect to the distance from the stimulation site, by starting from 4 electrodes (∼50 µm) until 30 electrodes (∼2827 µm) after stimulus. This indicates that the first spikes in case of fast responses (green) occur within (∼60-70 ms) after stimulation, regardless of their distance from the stimulus, whilst, first spikes in long-lasting responses (blue) begins at ∼85 ms, in close proximity to the stimulus (within 4 electrodes distance), and delayed to 120-140 ms in the distant ones.

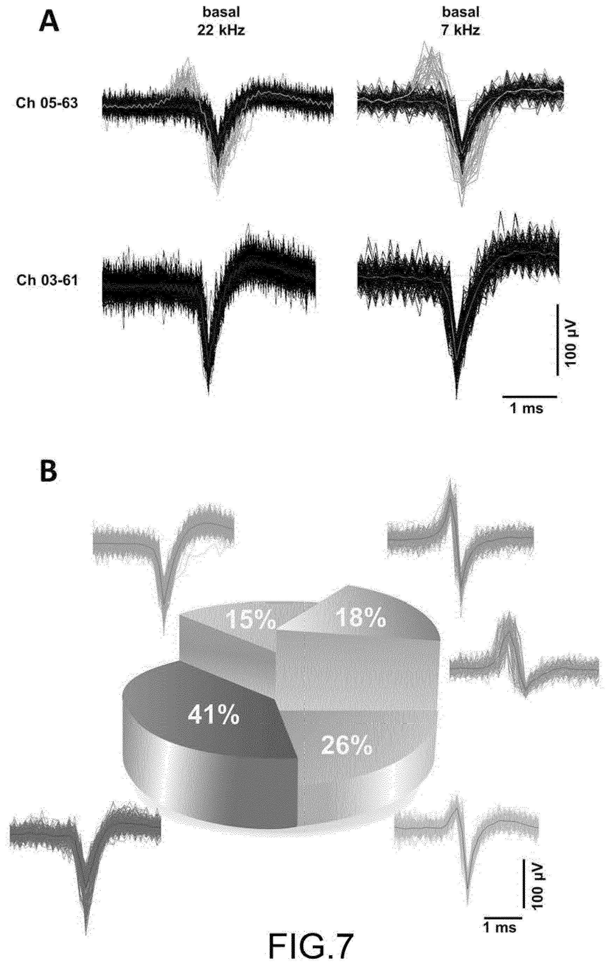

Figure 7 shows the Categorization of extracellular spike waveforms of iCell neuronal recorded. (A) Waveforms of extracellular spontaneous activity recorded with two sampling rates, namely 7 kHz and 22 kHz. No significant differences are noticed. The sorting of waveforms into units performed on different sampling frequencies, also show identical results, where exemplary multi-units are detected in ch 05-63 and only single-unit in ch 03-61. (B) Waveform classifications of evoked responses that are detected and sorted at 7.8 kHz sampling frequency. Two main classes were found; belonging to negative spikes namely (monophasic 41 %, biphasic 15 %, and triphasic 26 %), and positive biphasic spikes (18 %). The repertoire of evoked responses is in line with the physiological repertoire of evoked waveforms observed with murine cell cultures (Jimbo and Kawana 1992; Wagenaar, Pine, and Potter 2004; Ide et al. 2010).

### Experimental section

### Abstract

The recent availability of human induced pluripotent stem cells (hiPSCs) hold great promises as a novel source of human-derived neurons for cell and tissue therapies as well as for in vitro drug screenings that might replace the use of animal models. However, there is still a considerable lack of knowledge on the functional properties of hiPSC-derived neuronal networks, thus limiting their application. Here, upon optimization of cell culture protocols, we demonstrate that both spontaneous and evoked electrical spiking activities of hiPSC-derived neuronal networks can be characterized on-chip by taking advantage of the resolution provided by CMOS multielectrode arrays (CMOS-MEAs). These devices feature a large and closely-spaced array of 4096 simultaneously recording electrodes and multi-site on-chip electrical stimulation. Our results show that networks of hiPSC-derived neurons can respond to electrical stimulation with a physiological repertoire of spike waveforms after three months of cell culture, a period of time during which the network undergoes the expression of developing patterns of spontaneous spiking activity. To achieve this, we have investigated the impact on the network formation and on the emerging network-wide functional properties induced by different biochemical substrates, i.e. poly-DL-ornithine (PDLO), poly-L-ornithine (PLO), and polyethylenimine (PEI), that were used as adhesion promoters for the cell culture. Interestingly, we found that neuronal networks grown on PDLO coated substrates show significantly higher spontaneous firing activity, reliable responses to low-frequency electrical stimuli, and an appropriate level of PSD-95 that denotes an adequate neuronal maturation profile and synapses stabilization. However, our results also show that neither after three months of culture the network does achieve complete maturation.

Taken together, our results demystify the tight relationship existing between substrate coatings and emerging network properties, i.e. spontaneous activity, responsiveness, synapse formation and maturation. Additionally, our results provide a baseline on the functional properties expressed over three months of network development for commercially available hiPSC-derived neurons. This is a first step toward the development of functional pre-clinical assays for compounds testing on human-derived neuronal networks with CMOS-MEAs.

### Introduction

Along with the human world developing there has been a sequel evolution of diseases. In order to advance in the development of therapeutic approaches for these daunting challenges, novel methodologies able to accelerate research are needed. This is particularly crucial for brain diseases, where disease-specific therapies for many neurodegenerative disorders are still missing and a longer life expectancy is constantly increasing their societal impact. In this field, drug-discovery, therapeutic research and development (R&D) face major challenges that are also related to the lack in understanding of the normal brain functions and disease mechanisms. A proposed valuable way is the development of effective pre-clinical assays able to accelerate R&D on molecular targets. Toxicity and efficacy of molecular drug candidates are often issues in the clinical trials and are also arising from failure in translating results from drug trials obtained from animal testing to human. Consequently, the lack of attrition in the pre-clinical phase in drug discovery is a major obstacle in neuropharmacology, resulting in a high economic burden and in slowing down therapeutic developments (Orloff et al. 2009).

A recent giant step holding a great potential for drug discovery has been made by the development of induced pluripotent stem cells (iPSC) derived from human cells (Takahashi et al. 2007). In the recent years, the iPSC technology is attracting a growing interest for studying the pathogenesis of human diseases and for investigating their use to grow in vitro models for pharmacological and toxicological testing. In addition, these cells have a significant potential for research on specific pluripotent cell-based therapeutic approaches (Espuny-Camacho et al. 2013).

For both application fields of the iPS technology, i.e. cell-based therapeutic and in vitro compound testing, the electrophysiological properties of human-derived electrogenic cells need to be assessed. Different laboratories have been examining the electrophysiological responses of hiPSCs to pharmaceutical compounds by using conventional electrophysiological methods. In particular, studies on hiPSC-derived cardiomyocytes have been remarkably advanced in the recent years. Hence, as reviewed in (Rajamohan et al. 2013) nearly 60 compounds have been quantified for drug screening by using patch-clamping and conventional, 60 electrodes, multielectrode arrays (MEAs) to interrogate the electrophysiological properties and responses of these cultures. More recently, stable lines of hiPSC-derived neurons have become commercially available, here referred as (iCell neurons™), offering thus a unique opportunity for developing standardized in vitro assays for drugs targeting central nervous system (CNS) disorders. However, the network electrophysiology of these iCell neurons™ was so far only partially investigated. In this respect, the spontaneous activity that emerges during in vitro network development was characterized on-chip by using conventional multielectrode arrays recordings from 60 electrodes (Odawara et al. 2014). These devices represent a well-established technology that was used for decades to record network-wide extracellular activity from neuronal cultures prepared from rodents and to study the developing patterns of network activity (Wagenaar, Pine, and Potter 2006; Chiappalone et al. 2006) as well as responsiveness to electrical stimuli with network-wide elicited spiking activity (Jimbo and Kawana 1992; Wagenaar, Pine, and Potter 2004; Ide et al. 2010). In addition to spontaneous activity, a wealth of data describing the cellular, molecular, and functional properties of iCell neurons™ acquired with different methodologies has been reported (Whitemarsh et al. 2012; Haythornthwaite et al. 2012; Gill et al. 2013; Dage et al. 2014; Chatzidaki et al. 2015), while a consensus research on the properties of these neurons is still emerging.

In the present study, we deeply investigate the developmental electrophysiological properties of iCell neuronal networks formed in vitro as one of the hiPS cell lines that has been developed commercially to supply large pure quantities of differentiated human neurons.

By testing the effects of different cell culture conditions, that do not involve the use of glial co-cultures or conditioned media as previously reported (Odawara et al. 2014), the network development over three months is here characterized by means of electrical read-outs from large-scale networks by using an emerging generation of active MEAs (Figure 1), as well as optical read-outs from morphological fluorescence and confocal micrographs documenting their growth, function, and synaptic maturation. Both spontaneous activity and responsiveness to electrical stimuli of the network are studied. Given that during developmental stage, the brain is a receipt of external inputs that are critical to modulate excitatory and inhibitory synapses as a function of homeostatic synaptic plasticity (Turrigiano and Nelson 2004), electrical stimulation and recording capabilities through MEAs have been considered in previous works as adequate approaches for the experimental study of neuronal development and plasticity.

The active MEAs used in this work are realized with complementary-metal-oxide semiconductor technology (CMOS) and provide high spatiotemporal resolution electrical recordings consisting in sub-millisecond sampling of extracellular signals from the whole array of 4096 electrodes (L. Berdondini et al. 2001; L Berdondini et al. 2005; Luca Berdondini et al. 2009) as well as multiple distinct on-chip sites for electrical stimulation. In this respect, two configurations of CMOS-MEAs were used in this study, i.e. BioChip 4096S+ for recording the network spontaneous activity, and BioChip 4096E for recording the evoked responses of human derived neuronal networks (see Materials and Methods). As reported in our previous work on primary neuronal cultures from rodents (Maccione et al. 2010), the electrical read-outs provided by these active devices allow to reduce the undersampling of neuronal activity in networks, i.e. 4096 vs. 60 simultaneously recording electrodes, and to improve the statistical significance of mean network-wide activity parameters, such as the mean firing rate, for each recorded culture. Additionally, the high electrode density can also allow to detect a weak electrical activity indicated by a few spiking neurons within the network.

Importantly, in order to promote cellular adhesion, growth, and formation of functional networks of primary neuronal cultures as well as iCell Neurons™, appropriate biochemical substrates that consist in polycationic adhesion promoting molecules are needed. Here, we have therefore also investigated the use of three different coating reagents, i.e. poly-DL-ornithine (PDLO), poly-L-ornithine (PLO), and polyethylenimine (PEI), all supported with laminin, a component of the extracellular matrix that previously reported to promote neurite axonal growth (Matsuzawa et al. 1996). The effects of these biochemical substrates on the maturation and formation of synapse connections is here investigated by quantifying the expression level of PSD-95 protein and by its correlation with network activity changes obtained from activity recordings. Postsynaptic density-95 (PSD-95) is known as the major postsynaptic scaffold protein that is exclusively localized to glutamatergic synapses (Hunt, Schenker, and Kennedy 1996), and was reported to drive synaptic maturation (El-Husseini et al. 2000; Okabe et al. 1999), and to promote synapse stability (Ehrlich et al. 2007). Studies carried out in vitro have also previously shown that levels of PSD-95 increase during development (Q. Sun and Turrigiano 2011; Sans et al. 2000).

### Optical read-outs reveal synaptic formation and spontaneous activity during development of human iCell neuronal networks

Human-derived networks of iCell Neurons™ were grown on CMOS-MEAs for up to three months. In order to optimize the cell culture protocol, different adhesion-promoting molecules were tested by characterizing the cellular morphological development and network formation over development (see Materials and Methods).

Remarkably, after 24 hours, cells adhered on all coated substrates and started extending neuritic processes, as shown in Figure 2A. Indeed, iCell neurons™ exhibited better morphology on PDLO and PEI substrates even after 1 day in vitro (DIV); while cultures resulted more clustered, with fasciculated processes when plated on PLO substrates. Although we report here evidence for neuronal morphological differentiation on PDLO and PEI-coated surfaces, over the first two weeks in vitro, no major differences were observed on the morphological features after this period (up to a month), with the exception of a higher formation of clusters and fasciculated processes for cultures grown on the PLO-coated surfaces.

Next, to investigate the induced effects of the substrate conditions on the growth and iCell neuronal function, confocal micrographs of immunostained cultures were obtained over network development, for 3 time sets, i.e. at 8, 28, and at 90 DIV. Synaptic formation of a connected iCell neuronal network was examined by the quantification of expression of the major synaptic vesicle membrane proteins, i.e. synaptophysine-1, as well as markers for post-mitotic neural subtypes, composed by the inhibitory synapses, i.e. GABA transporter in the membrane of synaptic vesicles (V-GAT) and synthesizing GABA, the major inhibitory neurotransmitter in the CNS (GAD-65), and excitatory synapses, i.e. Glutamate transporter in the membrane of synaptic vesicles (V-GLUT), as demonstrated in Figure 2B. Moreover, a major microtubule associated protein was visualized with the MAP-2 marker and its expression showed a salient healthy neurite growth during developmental phases. Such a heterogeneity in neuronal networks is important to ensure physiological functional activity and it might be given as an intrinsic property of these human-derived neuronal cells that likely underpins their functional diversity among cultures (Llinas 1988). Additionally, confocal optical read-outs of c-Fos expression were used to quantify spontaneous activity in these cultures at cellular resolution for specific time sets. The expression of neuronal c-Fos marker suggested that cellular spontaneous neuronal activity can be detected already from 8 DIV and up to the entire experimental window of 90 DIV.

We then specifically investigated the effects of PDLO with respect to PEI on the maturation and stability profile of synapses in these human-derived neuronal networks. For this purpose, at 90 DIV, we performed immunostaining to assess the expression levels of PSD-95 in iCell neurons™ grown on CMOS-MEAs coated with the two aforementioned molecules as shown in Figure 2B. Notably, the fluorescent puncta of PSD-95 was reduced 2.5-fold in case of iCell neurons grown on PEI coated-surfaces compared to those grown on PDLO surfaces, as illustrated in Figure 2C and D, and also elaborated in low density human-derived neuronal cultures in Figure 3. Since PSD-95 proteins have been reported to play a central role in balancing the ratio of excitatory/inhibitory synapses (Prange et al. 2004), we also investigated the ratio of counted V-GLUT/V-GAT positive puncta obtained from iCell neurons at 90 DIV. Interestingly, we found that this ratio is (4.2 ± 0.28-fold) for neurons seeded on PDLO substrates, whereas it is (1.53 ± 0.1-fold) on cultures grown on PEI. This indicates a salient difference in inhibitory synapses for networks grown on the two coatings, as indicated by the higher expression level of V-GAT that leads to an imbalance of the ratio of glutamatergic/GABAergic synapses for networks grown on PEI-coated substrates, Figure 2E. In turn, this provides an evidence that PDLO is a very suitable surface functionalization for establishing a functional human derived neuronal network, while maintaining their physiological maturation and synaptic functional plasticity.

### Spontaneous electrical activity of human iPS derived neuronal networks undergoes developmental changes over three months of growth

### Developmental changes described by network-wide activity parameters

The CMOS-MEA chips enable to simultaneously record extracellular neuronal signals from a dense array of 4096 electrodes (see Materials and Methods), thus providing high-resolution recordings of spiking activity all over the network, as required for a precise quantification of mean activity parameters that characterize network-wide firing patterns over development (see Figure 1). In order to assess whether surface functionalization might induce different electrophysiological behaviors over the development of iCell neuronal networks, we evaluated the spontaneous activity over a time window ranging from 8 DIV and up to 90 DIV. In this respect, Figure 4A shows maps of activity over the 4096 electrode array that elucidate the developmental changes in the mean firing rate with respect to the first recorded phase at 8 DIV. Furthermore, Figure 4B shows some typical signal traces of the firing patterns recorded at different time-points over the three months of cell culture, while the counts of the detected spontaneous spikes and the number of electrodes recording spiking activity (i.e. active electrodes) are reported in Figure 4C & D. It can be observed that there is an oscillatory behavior in the spiking profile and a shift from random spiking activity that typically characterizes young cultures, to tonic network-wide spiking, manifesting bursting events at 81 DIV and stable burst firing patters for networks at 90 DIV. These developmental changes in the spontaneous activity suggest that the iCell neuronal network undergoes a maturation, similarly as what has been reported for murine primary cultures. However, the development of murine neurons requires much shorter time-window for their development (i.e. a few weeks) of network-wide activity (Wagenaar, Pine, and Potter 2006; Chiappalone et al. 2006). The developmental changes in the network-wide spiking activity mirror the evolution in the number of active electrodes that is preserved throughout the different stages of development, with the exception of a considerable sharp increase at 90 DIV for cultures grown on PDLO that is not observed for those grown on PEI. Indeed, our results show that iCell neurons™ begin firing action potentials at an early age (8 DIV), a time where the extracellular recordings are mainly composed by signals with single random spikes, with a fairly low number of active electrodes. At 81 DIV, the spiking activity substantially increased to reach its peak value and pursuits to a plateau at 90 DIV for PDLO cultures; whereas for PEI cultures, the peak is reached earlier at 73 DIV and it is followed by a significant decay in the firing rate at 90 DIV. It is important to note that neurons grown on PLO coated substrates did not exhibit any detectable extracellular spikes, notwithstanding the cellular activity indicated by c-Fos expression as shown in Figure 2B. This might be resulting from the bundled-like morphology of cultures grown on PLO-coated substrates, which impairs the neuron-electrode coupling by a weak cellule adhesion to the substrate, and finally, might lead to lift the cultured network upward. Thus, given the lack of activity and the morphology, cultures grown on PLO were washed-out after three weeks.

### Developmental changes unveiled by the distributions of the neuronal firing frequencies

The distribution of the iCell neuronal firing frequencies in the network was computed from the spontaneous extracellular signals simultaneously recorded at sub-millisecond resolution form the 4096 electrodes of the CMOS-MEA. This distribution can reveal the frequency contributions of all the differently spiking neurons in the developing network. Thus, it can be an indicator of the overall functional changes occurring at the cellular scale and goes beyond the solely monitoring of network-wide activity parameters by revealing changes occurring over time to both low-firing and high-firing neuronal populations that constitute the network.

As reported in Figure 5, our large-scale electrode array recordings show that iCell neuronal networks exhibit a lognormal-like distribution of neuronal firing frequencies, similarly as what has been reported for in vivo recordings on rodents, monkeys and human (Buzsáki and Mizuseki 2014) and, in our previous work, for in vitro murine neuronal networks (Amin et al. 2015). In order to quantitatively determine changes in the distribution occurring during network development, here we have computed the distribution for different time points, i.e. at 8, 81, and at 90 DIV, and we have used the mean parameter of the Gaussian fit. As shown in Figure 5A and inset, when human-derived iCell neurons™ grow on PDLO substrates, the mean parameter suggests that the distributions at 8 and 81 DIV tend to be nearly the same. This is followed by a significant 5-fold shift toward low firing rates at 90 DIV and an increase in the peak of the distribution. On the other hand, for networks grown on PEI substrates, the mean parameter of the Gaussian fit of spiking frequency distributions at 8, 81, and 90 DIV shows a tendency to maintain the same distribution with almost the same peak value, as in Figure 5B and inset.

### Human iCell neuronal networks can respond to electrical stimulation after three months of development

The ability of a network to respond to electrical stimuli, that depolarize neurons close to the stimulation electrode to fire action potentials and that successively elicit spiking activity in functionally connected neurons, is an important physiological property of a neuronal network. To the best of our knowledge, this property has not so far been reported for iCell neuronal networks, neither described using MEAs.

### Human iPS derived neuronal networks respond to electrical stimulation

For different ages of the iCell neuronal culture, trains of low-frequency electrical stimuli at 0.2 Hz were applied sequentially from the 16 stimulation electrodes integrated on the CMOS-MEAs. These electrodes are dedicated to electrical stimulation and are interleaved within the 4096 recording electrode array. In addition, pre-stimulation and post-stimulation spontaneous network activities were recorded.

We have observed that only neurons grown on PDLO coated substrates and exclusively at 90 DIV were capable to elicit spiking responses to electrical stimuli. Samples of evoked signal traces are shown in Figure 6A. Interestingly, due to the electrode embedded morphology, the stimulation artifact is confined to < 20 µm distance from the stimulation electrode, thus allowing to record spike evoked responses within 7 ms latency after stimulation, i.e. < 100 µm, and to observe propagating responses in the network. Under our cell culture conditions, these responses consist in different spatiotemporal patterns of spikes that appeared depending from the different stimulation sites. Our results show two major forms of electrically evoked network responses that can be distinguished from the rastergrams and from the post-stimulus-time-histograms (PSTHs) reported in Figure 6B. A first form consists in fast spiking responses of the network lasting for ∼100 ms after the stimulation, (e.g. stimulation electrode S7) followed by a network activity that returns to the pre-stimulation activity baseline. These responses are similar to those observed in murine primary neuronal cultures (Jimbo and Kawana 1992). A second form of responses was observed for stimuli delivered from some electrodes (e.g. stimulation electrode S12) and consisted in long-lasting spiking responses up to ∼500 ms in delay, after the stimulation instant. To the best of our knowledge, this second form of evoked responses has not been observed in primary murine neuronal cultures and might be the result of either the cell-type heterogeneity of developing networks or of an intrinsic property of these human iCell neurons™.

To further characterize the evoked spiking responses, we have also investigated the delays of the first evoked spikes after stimulation, by taking into account the two forms of responses, i.e. fast and long-lasting. The Figure 6C shows an overall average of the first spike latency for stimuli delivered by different stimulation sites, as recorded regardless of the distances of the recording electrodes. First, spike latencies were quantified as lasting ∼70 ms for fast responses and ∼120 ms for long-lasting responses. Secondly, we have tested the relationship between first spike latency and the distance from the stimulating electrode, by considering that this distance can be maximally of ∼2827 µm in a radius of 30 electrodes (see Figure 6D and Methods section). This analysis reveals the two forms of evoked responses that correspond to more pronounced prominent occurrence delays for the long-lasting responses.

Furthermore, to evaluate whether low-frequency stimulation affects the iCell network activity, the number of active electrodes and the mean firing rate were computed for pre- and post-stimulation recordings. The number of active electrodes increased slightly, i.e. from 564 ± 28 to 688 ± 21, before and after stimulation, respectively. This is accompanied by a slight decrease in the mean firing rate (spk/s), i.e. from 0.66 ± 0.03 to 0.58 ± 0.03, before and after stimulation, respectively. Notably, despite these slight changes, no evidence for clear changes induced by the electrical stimuli occurred in the network features, thus suggesting no considerable plasticity being induced by low-frequency stimuli into these human derived neuronal networks.

### Analysis of the evoked spike waveforms shows that human neuronal cultures exhibit a normal repertoire of physiological extracellular spike signals when responding to electrical stimuli

The electrically evoked spike waveforms were analyzed in order to describe the repertoire of extracellular spike waveforms of iCell neuronal networks and for comparison with those reported for primary neuronal cultures from rat or mouse embryos. To do so, we have used a spike sorting algorithm included in the Offline Sorter (from Plexon Inc., USA) (see Materials and Methods). Given the relatively low sampling frequency, i.e. 7.8 kHz/electrode, of our electrode signals when CMOS-MEAs are operated to record from the whole array of 4096 electrodes, we have at first assessed the sorting performances. To do so, we have compared spike sorting results obtained from the same culture and the same electrode area with extracellular signals sampled at the normal frequency, i.e. 7.8 kHz/electrode, and at a higher frequency of 22 kHz/electrode. This was made possible since CMOS-MEAs act as imaging sensors that allow to select a region of interest, i.e. an area of the electrode array, and thus to increase the sampling frequency in this recording modality. Details on the approach are reported in the Materials and Methods. It has to be highlighted that high sampling frequencies are particularly important for spike sorting in vivo recorded extracellular signals, but might not be needed for cell culture signals given the much lower cellular density and bi-dimensional organization of the network. This consideration was confirmed by our results. Indeed, as shown in Figure 7A the spike sorting results for the same network area and for both sampling frequencies do not show significant differences in the number of sorted units and in the features of the spike shapes. In particular, results show that most of the electrodes record single-units (> 95% [n=3]) and only a very few electrodes record two units (< 5% [n=3]). Following this, we have then categorized the repertoire of spike waveforms expressed by iCell neurons™ upon recording at 7.8 kHz/electrode from the whole array of 4096 electrodes, as shown in Figure 7B. As reported for embryonic neuronal cultures from rats (Nam and Wheeler 2011), categorization of the waveforms was done based on the amplitudes of the positive and negative peaks of the sorted spikes. Similarly as in the Nam and Wheeler paper for murine cultures, we found that the majority of the evoked spike waveforms (82 %) are negative spikes, i.e. featuring a larger amplitude for the negative peak, while only a small fraction of spikes are positive (18 %), i.e. with a larger amplitude for the positive peak. Further categorization indicates that 41% of the spikes are monophasic negative events, 15% are biphasic negative spikes, and 26% are triphasic negative spikes characterized by a small amplitude positive peak that is followed by a large amplitude negative peak and by a third small amplitude positive peak. The positive spikes were only biphasic waveforms, with a large amplitude positive peak followed by a negative peak. Interestingly, these results on sorted spike waveforms show that human neuronal cultures exhibit very similar extracellular waveforms as reported for physiological extracellular signals observed for embryonic primary neuronal cultures from mice or rats.

### Materials and Methods

### Preparation of hiPSC-derived neuronal cultures on CMOS-MEAs

Frozen stocks of hiPSC-derived neurons were obtained from (Cellular Dynamics International (CDI), USA) in vials containing at least 2.5 million plateable cells. Cells were thawed following the manufacturer's instructions and seeded at a density of 200'000 cells per CMOS-MEA chip (BioChip 4096S+ and 4096E, 3Brain GmbH, Switzerland). The BioChip 4096S+ integrates an array of 4096 recording electrodes (21 x 21 µm2 in size, 42 µm pitch) on an active area of (2.67 x 2.67 mm2) centered in a working area of (6 x 6 mm2). While the BioChip 4096E, integrates two interleaved arrays of 4096 recording electrodes (21 x 21 µm2 in size, 81 µm pitch) and 16 stimulation electrodes (21 x 21 µm2 in size, 1246 µm pitch) on an active area of (5.12 x 5.12 mm2) centered in (6 x 6 mm2). For the cellular growth, three different adhesion factors were tested for coating the CMOS-MEA surface, namely poly-DL-Ornithine (PDLO) 50 µg/ml (Sigma P0671), poly-L-Ornithine (PLO) 0.01% (Sigma P4957), and polyethylenimine (PEI) 0.1% (Sigma P3143), all dissolved in borate buffer (100 mM boric acid, 25 mM sodium tetraborate at PH 8.4) (all from Sigma-Aldrich, Italy). In brief, CMOS-MEAs were sterilized in 70% ethanol for 20 minutes, followed by bountifully four times washing with Double Distilled Water (DDW), then chips were preconditioned, i.e. incubated for 48 hour with Complete Maintenance Medium. Afterwards, medium was aspirated and chips were immediately coated and incubated for 6 hours at 37°C with 5% CO2, 95% O2 and saturated humidity. The adhesion factor was aspirated after incubation and each chip was rinsed 4 times with DDW and left overnight to dry under a sterile laminar flow. A vial containing 2.5 million plateable iCell neurons™ was placed in a 37°C water bath for exactly 3 min. Neurons were then transferred into a 50 ml centrifuge tube, and 1 ml of Complete Maintenance Medium was added slowly (drop-wise) to the cells. The Complete Maintenance Medium containing iCell maintenance and supplement media; (from CDI, USA) in addition to 1% penicillin-streptomycin (Life Technologies, Italy). Cells were swirled gently and a further 8 ml of complete maintenance medium was added slowly to the centrifuge tube. Finally, Cells were re-suspended at 5'000 cell/µl and 40 µl drop was seeded on each CMOS-MEA, this included 10 µg/ml of laminin (Sigma-Aldrich, Italy) that was added to the 8 ml cell solution. The chips were incubated at 37°C with 5% CO₂ and 95% O₂ and after 24 hour, 100% cell culture medium was exchanged with Neurobasal Complete Medium that is comprised of (1x Neurobasal-A (NBA), 10% KnockOut Serum Replacement (KSR), and 1% penicillin-streptomycin (all from Life Technologies, Italy). Seeded CMOS-MEAs were afterward incubated at 37°C with 5% CO2 and 95% O2 for up to 12 weeks where a 50 % medium exchange with Neurobasal Complete Medium was made every 4 days.

### Long-term, large-scale electrode array recordings and analysis of spontaneous activity

Extracellular electrophysiological activity of in vitro spontaneously active hiPSC-derived neuronal networks grown on CMOS-MEAs was monitored over several months by using a custom platform build with components of the BioCam system (3Brain GmbH, Switzerland). Multiple recording sessions of 10 minutes were performed at full-frame resolution (at 7.8 KHz/electrode from 4096 electrodes), in addition to recordings from a selected region of interest (ROI) (at 22 KHz/electrode from 1024 electrodes). Experimental data (acquisition rate of 60 Mbyte/sec) were stored locally or on a data server for further offline analysis. Spike detection analysis was performed by employing the Precise Timing Spike Detection (PTSD) algorithm (Maccione et al. 2009) integrated in the Brainwave software application (3Brain GmbH, Switzerland). A threshold of 9 times the standard deviation was used for spike detection, whereas burst events were identified if at least five consecutive spikes in an Inter Spike Interval (ISI) lower than 100 ms were detected. In our analysis, we considered only electrodes recording spikes with rates between 0.05 and 10 events/sec (i.e. "active electrode"). All spike trains were exported from BrainWave to Matlab (The MathWorks Inc., USA) files and were analyzed with custom Python scripts (Python Software Foundation. Python Language Reference, version 2.7 available at http://www.python.org), in particular for first order statistics of network-wide mean activity parameters, i.e. mean firing rate, active electrodes, and for the analysis of the neuronal firing frequencies distributions, where lognormal-like distributions and fit statistics were done as described in (Amin et al. 2015).

### Multi-site electrical stimulation on CMOS-MEAs and analysis of evoked responses

The hiPSC-derived neuronal networks were electrically stimulated with the on-chip integrated stimulating electrodes of the CMOS-MEAs chip. In our custom experimental platform, the 16 stimulation electrodes were connected to a 16-channel electrical stimulator (Plex-Stim Electrical Stimulator 2.0 System, Plexon Inc., USA) controlled with a custom software tool developed in C# programming language. Low-frequency (0.2 Hz) trains of biphasic current stimuli (300 µs per phase, positive phase first, peak-to-peak amplitude of 300 µA) were delivered for 3 min to all the 16 stimulation electrodes using a random sequence for the stimulation sites applying each stimuli (i.e. providing 35 stimuli for each electrode channel). As for spontaneous activity recordings, the 4096 recorded extracellular signals were exported and analyzed with custom Python scripts (Python Software Foundation). The electrically evoked responses were isolated from the recordings by binning the overall network spiking activity with a bin size of 25 ms. The detection of the stimulation artifacts was afforded with a hard threshold (Dayan and Abbott 2005). Afterwards, the evoked responses corresponding to 35 repetition of a stimulus for each site were aligned to the time zero and binned with 25 ms bin size. The post-stimulus-time-histograms (PSTH) (Gerstein and Kiang 1960) was computed from these isolated and aligned responses. The first 10 ms after the stimulus were discarded in order to avoid any possible effect of the stimulus artifact. The PSTHs were computed for each stimulating electrode by computing the average, the lower and uppermost percentiles over all evoked activities for each time bin. Additionally, to characterize the two forms of network evoked responses, i.e. fast and long-lasting, we computed the timing of first occurring spikes. First, globally, we accumulated statistics on latencies of first spikes in response to each train of 35 stimuli from all stimulating sites. Secondly, this approach was extended to test the effect of the distance from the stimulating site on the first spike occurrence in 500 ms time window by considering a circular region spreads from the close proximity of the stimulus, i.e. 4 electrodes (∼50 µm), to the distant ones, i.e. 30 electrodes (∼2827 µm), spacing 2 electrodes (∼12.5 µm). Responses were considered reliable for computation when electrodes provided responses to at least 10 out of 35 low-frequency stimulation trials.

### Analysis of spontaneous and evoked spike waveforms

Spike waveforms were analyzed in a similar way as previously reported method (Nam and Wheeler 2011) and based on a subset of parameters characterizing the spike shape. Also for this analysis, the spike detection was performed with the BrainWave acquisition software using the PTSD algorithm. The artifact detection was applied for these electrically evoked datasets. The spike waveforms were exported as Matlab files (time windows of 4 ms, centered on the peak of the spike) and, in order to sort the spike waveforms with the Offline Sorter™ (Plexon Inc, USA), these files were converted into .nex files with a custom Matlab® script (MathWorks, USA). Spike sorting was performed with a t-distributions expectation-maximization (T-dist E-M) algorithm (Shoham, Fellows, and Normann 2003) including a unit range of 1 to 3.

### Immunofluorescence protocol

Culture medium was removed and cell culture washed using phosphate-buffered saline (PBS) at 37°C, then cells were fixed in paraformaldehyde (4% in PBS-1X) for 15 minutes at room temperature (RT). Following fixation, cells were washed gently 4 times with PBS-1X and then cells were permeabilized with 0.1% Triton in PBS-1X (PBST) for 10-15 minutes and blocked with 5% Normal Goat Serum (NGS) (EuroClone S.p.A, Italy) for 1 hour before incubation with primary antibodies. Cells on CMOS-chips were then incubated overnight at 4°C in primary antibodies diluted in the blocking buffer. The following primary antibodies were used; guinea pig anti-MAP2 (1:1000), mouse anti-Synaptophysin-1 (1:200), rabbit anti-V-GAT (1:500), guinea pig anti-V-GLUT 1 (1:1000), mouse anti-GAD65 (1:500) (all from Synaptic Systems, Germany), as well as rabbit anti-c-Fos (1:500) (Calbiochem Millipore, Italy), and mouse anti-PSD-95 (Thermo Scientific, Italy). Progressive 3 washing steps in PBST for five minutes per wash were performed, and cells were incubated for 1 hour in the dark at RT with the corresponding secondary antibodies including; Alexa Fluor 488, Alexa Fluor 546, and Alexa Fluor 647 (all from Life Technologies, Italy). Afterward, cells were washed 4 times with PBST and incubated for 15 minutes in the dark at RT with the nuclear marker Hoechst 33342 (1:500) diluted in PBS-1X (ThermoFisher Scientific, Italy). Images from iCell neurons™ were acquired and visualized with 20X and 40X objective lenses using Leica SP5 upright confocal microscope (Leica Microsystems, Germany), Nikon A1 inverted confocal microscope (Nikon Instruments, Italy), and a BX61 Olympus fluorescence microscope (Olympus Life Science Solutions, Italy).

### Image analysis

In order to quantify the PSD-95 assembles in various densities of human neuronal network coupled to CMOS-chip, we performed the granulometric filtering (Prodanov, Heeroma, and Marani 2006) method on the confocal micrographs. Images were converted to greyscale to enhance the contrast between signal and noise, followed by setting a manual threshold. Afterward, small grains of PSD-95 construct, consisting of several pixels, were identified based upon their sizes and shapes in a heterogeneous background image. Integral intensity thresholding then was used to assign the granulometric filtering intensities and performed using Imagej analysis software platform (Schneider, Rasband, and Eliceiri 2012). The blue and red cross-sections display the fluorescence and the corresponding filtered intensities, respectively (see Figure 2C and Figure 3). Arbitrary offset, i.e. dashed line at 0.07, was defined to pinpoint the PSD-95 puncta above that level while the yellow bars represent the location of the puncta above the offset. On the other hand, PSD-95 expression construct, V-GLUT, and V-GAT densities were performed using automatic particle counting integrated in Imagej platform. The synaptic density was hence, analyzed by counting immunoreactive puncta per µm2 in at least 9 visual fields, randomly chosen in multiple cultures (n=3), measuring ∼ 79 x 79 µm from the 1024 x 1024 pixel image, where only fluorescence puncta that had their major axes within 0.3-1.3 µm were selected for analysis (Ziff 1997).

### Statistical analysis

All data are expressed as the mean ± standard error of the mean (SEM). Differences between groups were generally examined for statistical significance using one-way ANOVA analysis with a cutoff at p ≤ 0.05 for significance. Furthermore, a non-parametric Kruskal-Wallis method was used for data not normally distributed.

### Discussion

Viable neuronal networks should express developing patterns of spiking activity during their growth in vitro and should respond to electrical stimuli with network-wide elicited spiking activity (Jimbo and Kawana 1992). These are fundamental properties of cultured neuronal networks as known from several decades of research on neuronal networks prepared from rodents. However, to the best of our knowledge these properties were still not completely demonstrated for cultured networks of human pluripotent stem cells (hiPSC) derived neurons, a young field of research that needs to further explore the electrophysiological properties of these human-derived neuronal networks. Here, upon optimization of the cell culture conditions, we show that networks of a commercially available line of hiPSC-derived neurons can indeed express all these physiological network-wide electrophysiological properties when grown for three months in vitro. By applying an emerging large-scale multielectrode array technology (CMOS-MEAs) enabling long-term, i.e. several months, simultaneous recordings from a dense array of 4096 electrodes and electrical stimulation with on-chip electrodes. The relevance of this characterization and of the successful use of CMOS-MEAs is related to the potential applications of human-derived neurons. Indeed, human pluripotent stem cells (hiPSCs) are an emergent and promising source of human-derived neurons for pre-clinical research and cell-based assays, for CNS-drugs and for investigating brain diseases mechanisms on human-derived neuronal networks. So far, these neurons were demonstrated to exhibit a range of physiological and pathophysiological properties of native human neurons, under healthy or neurodegenerated conditions. However, in order to advance with the application of these human-derived neurons, it is necessary to optimize and standardize the cell culture conditions and to characterize the electrophysiological properties emerging form networks that these neurons can form. To investigate the electrophysiological properties of a network with cellular details and over several weeks (or months) of development, conventional single-cell electrophysiological techniques as well as non-label-free imaging methods are not adequate. Thus, alternative methodologies that enable non-invasive long-term measures are needed, both for studying the network electrophysiology as well as for the development of in vitro assays based on human-derived neurons. As demonstrated in this work, CMOS-MEAs and the so provided high-resolution electrical read-outs represent a valuable, high-information content candidate neurotechnique that can be extended in the near future to multi-well configurations in order to improve the read-out throughput. In particular, multielectrode arrays provide high quality simultaneous extracellular recordings of spiking activity from a large number of neurons that constitute the network. Extracellular electrode recordings do not alter cells and are an ideal methodology for long-term recordings of several weeks. In addition, the performances of recently developed CMOS-MEAs allow to record spikes from several thousands of neurons simultaneously. Such an extensive recording of spiking activity within cultured neuronal networks was previously demonstrated in our work by highlighting the useful improved statistical significance of network-wide activity parameters already with a very few parallel trials (Maccione et al. 2010).

Here, bi-directional CMOS-MEAs (i.e. with recording and electrical stimulating electrodes) were used to grow and to monitor over three months the spontaneous spiking activity emerging from human-derived neuronal networks and to assess their responsiveness and response properties to multi-site electrical stimulations. Since the optimization of the cell culture conditions is a critical aspect for standardized assays, in this work we optimized the cell culture protocols to grow on CMOS-MEAs viable human-derived neurons for several months. Remarkably, differently than previously reported results (Odawara et al. 2014), we found that astrocyte-neuron co-cultures are not mandatory to achieve network growth and maturation, rather, an optimized surface functionalization utilizing PDLO is found to be a prerequisite.

Developmental changes in the spontaneous activity of human-derived networks, prepared with the same iCell neuron™ line as used in this work, were previously described with recordings obtained from low-resolution multielectrode arrays of a few tens of electrodes (Odawara et al. 2014). However, these conventional MEAs require to perform multiple parallel experiments in order to ameliorate the low statistical significance of mean activity parameters for each culture and miss the information content on single-neuron activity that can be provided by the dense electrode array integration of CMOS-MEAs.

### Poly-DL-Ornithine coated surfaces result promoting enhanced morphological and functional development of human derived neuronal networks

Surface functionalization of multielectrode arrays is a two-fold problem, with relevant implications to the culture viability and to the electrical recording performance. Indeed, the neuron-electrode interface is a critical aspect for the development of viable and functional electrode-based neural devices (Wheeler et al. 1999; Jungblut et al. 2009; Y. Sun et al. 2012) as needed to study the activity in neuronal networks as well as their responses to pharmacological or electrical stimuli. In this study, this well-known issue for rodent primary neuronal cultures was even more stringent given the use of recently developed human-derived neurons and of a non-standard cell culture substrate. Therefore, we experimentally investigated the morphological and functional effects induced by three different biochemically active molecules deposited on the chip surfaces before cellular seeding and we have evaluated their ability to support cellular attachment, long-term culture viability and to enhance neuroelectronic coupling. In particular, iCell neuronal cultures were grown on the CMOS-MEA and on glass surfaces coated with PDLO, PEI, and PLO. We quantified the growth of these cultures with the use of fluorescence and confocal microscopy for up to 90 DIV even though morphological differences have already been qualitatively noticed already after 1 DIV. Morphologically, surfaces that were coated with PDLO and PEI showed homogeneous cellular growth and maintained distributed individual cells, while cultures grown on PLO coated substrates showed a strong prominence to the formation of cellular clusters and fasciculation of the neuritic processes. In addition, networks grown on PLO coated CMOS-MEAs did not show spontaneous activity and neither evoked responses, despite the high-electrode density that is expected to allow for recordings also in case of a very few spiking neurons. On the other side, spontaneous electrical activity was recorded from CMOS-MEAs coated with PDLO or PEI, with significant differences induced by the two coatings during development. However, evoked responses to electrical stimulation were recorded only from PDLO-coated devices. A reason of this particular functional feature of the PDLO might be conferred by, first, the additional carbonyl groups which contribute negative charges, and secondly, to the structure of ornithine molecules built up in a high molecular weight of the polycationic organic substance, thus providing sufficient binding sites for cell attachment.

On the other hand, responsiveness to electrical stimuli was observed only at 90 DIV and appears to be related to the PDLO-induced network growth. Since synaptic transmission prevails the cellular communication between neurons and it is required for brain function, hence, stabilization of synapses and orchestration of the pre- and postsynaptic terminals are critical to synaptic development, plasticity and thus neuronal dynamics, as conferred by hardwired neural networks. In light of this, we thus examined the formation and stabilization of iCell neuronal synapses obtained by the underlying coated substrates. This was done throughout the quantification of the PSD-95 expression level. PSD-95 is a membrane-associated protein predominant in the postsynaptic density that is implicated in maturation, synaptic strengthening, and plasticity. Consequently, our result pinpoints, after 90 DIV of network development, the balance between excitatory and inhibitory synapses (as indicated by the V-GLUT/V-GAT ratio for human neuronal ensembles grown on PDLO and PEI coated substrates); a critical ratio for determining neuronal excitability (Prange et al. 2004). Confocal image analyses with a granulometric filter (Prodanov, Heeroma, and Marani 2006; van den Bogaart et al. 2011) show that PSD-95 is highly expressed in neurons grown for three months on PDLO coated substrates and it is significantly lower for neurons grown on the PEI coated ones. Since PSD-95 promotes the stabilization of young synapses (Taft and Turrigiano 2014), our data suggest a developmental regulated process that results in more mature and stable young synaptic contacts for neurons grown on PDLO surfaces, albeit their still immature functional properties. Rather, a compromised maturation profile of neurons maintained on PEI substrates is observed. Previously reported evidences converge on the fact that appropriate expression of PSD-95 enhances synaptic clustering of α-amino-3-hydroxy-5-methyl-4-isoxazole propionic acid receptor (AMPAR), i.e. GluR1 subunit, that in turn increases excitatory presynaptic input (El-Husseini et al. 2000) as demonstrated in our data by V-GLUT quantifications. In addition, it should be noted that two neuronal classes constitute the cerebral cortex, where -80% are excitatory glutamatergic neurons and ∼20% are inhibitory GABAergic interneurons (Wonders and Anderson 2006). Thus, our results suggest an effect by which the ratio of excitatory to inhibitory synapses (V-GLUT/V-GAT) is lower in neuronal ensembles grown on PEI coated substrates (i.e. ∼60% V-GLUT and ∼40% V-GAT), due to an increase in inhibitory and a decrease in excitatory synapses, possibly favoring inhibition, compared with a higher ratio for neurons grown on PDLO coated substrates (i.e. ∼80 % V-GLUT and ∼20% V-GAT), thus with excitation preference. If the increase of V-GAT expression in neurons grown on PEI, rather than PDLO, is physiological relevant, this could be associated with other scaffolding proteins and cell adhesion molecules (Levinson and El-Husseini 2005) that are beyond the aim of this work and that can be an object of future investigation.

To the best of our knowledge, this is the first report demonstrating the relationship that exists between surface treatments, network morphology and electrophysiological activity of human iPS derived neurons. These effects were observed for glass coated substrates as well as on CMOS-MEAs and are thus not related to the surface topology of CMOS-MEAs that emerges from the multiple layers of the integrated electrical circuits. In our opinion, these observations might contribute in the development of standardized protocols to grow human-derived neurons and for electrical-read outs for functional screenings.

### Human-derived neurons coupled to CMOS-MEA exhibit spontaneous activity patterns that undergo developmental changes during three months of culture

By using CMOS-MEAs, we demonstrated that human iCell neuronal networks display complex patterns of extracellular spontaneous activity at different developmental days (from 8 to 90 DIV). Notably, these high-resolution recordings show that in an early stage of network formation, the network-wide firing patterns are characterized by sparse single spikes from a few neurons; successively evolve during network development toward tonic network-wide firing manifesting bursting events at 81 DIV and that stabilize by displaying large bursting events propagating in the network at 90 DIV. Importantly, these recordings were obtained without the support of astrocyte co-cultures as it was previously reported (Odawara et al. 2014). Instead, the neuron-electrode coupling and neuronal maturation properties were enhanced by the use of PDLO-coatings of the substrate. By looking at the firing rate at 90 DIV, it is striking to observe a much higher activity for neurons grown on PDLO coated substrates compared to a suppression of the activity for the PEI coated ones. This suppression can be associated with the significant decrease in the PSD-95 expression, which might be the result of differences in synapse stabilization and formation rates, variation in synapse lose rates, or both (Okabe et al. 1999). It should also be remarked that the high spiking activity observed in PDLO coated samples, combined with a prominent synchronous activity, i.e. synchronous bursts, promotes by itself the stabilization and the growth of newly formed synapses during development (Ben-Ari 2001). Yet, it is also worth noting that our experiments were performed in neuronal networks that, albeit the long incubation period of 90 DIV, could be still relatively immature, as it has been previously reported based on the gene expression profile of iCell neurons™ observed in neonatal prefrontal cortex (Dage et al. 2014). To that end, our results suggest that we might have underestimated the developmental time needed to obtain functional and mature human derived neuron ensembles.

Originally, large-scale recordings of neuronal spiking activity allow to compute the firing frequency distributions for different time-points, i.e. 8, 81, and 90 DIV, over the three-month long in vitro culture. Results show skewed lognormal-like distributions for each time-point and for two different substrate coatings. These distributions are shifted or concomitant depending on the maturation of the network, either on PDLO or PEI coated substrates. Such lognormal-like distribution of the neuronal firing frequencies was recently reported for in vivo neuronal recordings in different brain circuits, on monkeys and rodents (Buzsáki and Mizuseki 2014) and also in our previous study using rat cultures coupled to 4096-electrode arrays (Amin et al. 2015). Remarkably, here our results show that such distribution is observed also in vitro on human-derived neurons. This finding experimentally confirms that the lognormal-like distribution might be a physiological attractor for the spikes contributions of neurons within a network also in vitro. Additionally, as suggested by (Teramae and Fukai 2014), changes in distributions observed during development might be linked to changes in the distribution of the synaptic weights and might underlie activity-dependent synaptic changes during development. Furthermore, another possibility for the source of asymmetrical distribution of firing rate reported in cultures grown on PDLO vs. PEI, might be due to the imbalance in the excitatory and inhibitory synaptic drive during network activity (Yassin et al. 2010). This hypothesis is further supported by our finding on the (V-GLUT/V-GAT) ratio that indicates such a balancing difference between networks grown on PDLO-/PEI-coated substrates.

### Human derived-neurons respond to electrical stimulation

Finally, responsiveness to electrical stimulation is expected in neuronal networks, as largely investigated over the last decades with multielectrode arrays in vitro neuronal cultures prepared from rats or mice. We found that only iCell neuronal networks grown on PDLO coated substrates can respond to electrical stimuli with network-wide responses, but only after three months of culture. This might denote a long cell culture time needed by these neurons to establish effective functional connections among neurons. In addition, we also observed that two classes of evoked responses were distinguishable, either lasting less than 100 ms, or, surprisingly, lasting more than 500 ms for some of the on-chip stimulations sites. In our experience, such a long-lasting response to electrical stimuli was not previously observed on cultures of rodent's neurons, either from wild-type mice or rats. These long-lasting responses might result from the heterogeneity in development of the effective connectivity in the same network; hence, causing a delay in the neuronal information flow conveyed thoroughly evoked spiking activity. Another reason for these long-lasting responses might be related to recurrent activations of synaptic excitation (Lau and Bi 2005) and might underlie the dynamical properties of a subset of the network that provides to some extent a specific subpopulation of neurons in the same network. Finally, it is also conceivable that this type of responses might indicate typical features of human neuronal networks that provide their own importance and function for the maintenance of the physiological conditions during development.

On the other hand, the lack of responsiveness to electrical stimuli over three month of culture grown on PEI substrates is still unclear and might be due to the physiological properties of this line of human-derived neurons, when they are seeded on PEI surfaces, as well as it has been shown that human iCell neurons™ are developing slower than what is observed for rat or mice neurons (Ohara et al., 2015). Alternatively, the need of a long maturation, might be, to a certain extent, due to the effect induced by the lack of astrocytes in these cultures, as well as nutritive components and serum protein content in the supplemented media (Dodla, Mumaw, and Stice 2010). Additionally, human neurons used in this work were prepared from pre-differentiated cryopreserved stocks which may require a longer recovery period to develop a physiological neuritogenesis compared to those prepared from fresh rodent tissues.

Finally, our analysis of the evoked waveforms shows that the repertoire of the electrically evoked signal responses for human-derived neurons does not show significant differences with respect to the one observed for cultured hippocampus networks from rat embryos.

### Conclusions and Outlooks

The assessment of the functional properties emerging from human-derived neuronal networks and their cell culture conditions optimization are two major requirements for the exploitation of this emerging biotechnology into a wide range of applications, in particular toward cell-culture assays in vitro for drug screenings and toxicology.

In this respect, in the present study we have demonstrated the value of a combined and optimized use of two emerging technologies, namely hiPSC-derived neuronal cultures and large-scale electrode array platforms (i.e. CMOS-MEA). Importantly, our morphological and electrophysiological results allowed us to pinpoint the key role of surface functionalizations for significantly enhancing morphological and functional properties of human-derived neuronal networks grown in vitro up to several months. Additionally, by means of simultaneous on-chip recordings from 4096 electrodes, this optimization of the cell culture conditions allowed us to further demonstrate that networks of human-derived neurons can express both network-wide spontaneous activity patterns that change over development and electrically evoked responses, as it can be expected from viable networks. However, despite the unique value of the human derived neurons used in this work, our results also highlight that significant challenges on human-derived neurons remain to be overcome and need to be further investigated. Indeed, even after three months of culture and optimization of the cell culture conditions, our results show that the emerging network properties are not completely comparable with what we know from studies on neuronal networks prepared from mice or rats neuronal cultures. This might be interpreted as an unmet sufficient level of maturation of the network or as intrinsic functional properties of networks formed by these human-derived neurons. Based on our experience as well as on results presented in this study, we consider the first hypothesis as the most plausible. In light of this, new in vitro differentiation strategies and protocols of hiPSCs have to be advanced, given their role to generate more stringent functionally differentiated neurons for their final appropriate use in research and therapy.

Notably, neuronal responsiveness to pharmaceutical compounds has been reported to be maturation-state-dependent (Falcão et al. 2006); therefore it is crucial to profoundly characterize the maturation profile of these human derived neurons in order to increase their responses fidelity and reproducibility for any potential pharmacological and toxicological testing. Thus, perspective studies must be performed over long-time (several months), to investigate the maturation profile of these human derived neurons. In this context, studies on hiPSC derived neurons; to quantify the expression of two GABA transporters are needed. These transporters, Na+-K+-2Cl-co-transporter (NKCC1) and the K+-Cl-co-transporter (KCC2) play a crucial role in regulating the intracellular chloride of neurons during developmental stages (Ben-Ari 2002). Thus, in our lab, studies on the expression of these transporters in iCell neurons™ are in progress and might contribute to determine a possible developmental GABA switch as well as to better understanding the functional properties and level of maturation of these neurons.

Overall, the results of this work represent in our opinion a relevant step toward the application of these human-derived neuronal networks for pre-clinical research and provide a baseline characterization of their functional development. Additionally, we demonstrate a powerful non-invasive platform for potentially developing effective assays with high-information content electrical read-outs that will require only minimal amounts of human-derived cells, biochemical and drugs. By way of extension, this platform, further developed from single-wells to multi-wells configurations, and the methods presented here might be used in the near future to screen for disease therapeutic applications on brain disease human neuronal culture models as it has been recently made available by the same company providing "MyCell disease and diversity products" and as it is under development in different laboratories.

### References

- Amin, Hayder, Alessandro Maccione, Stefano Zordan, Thierry Nieus, and Luca Berdondini. 2015. "High-Density MEAs Reveal Lognormal Firing Patterns in Neuronal Networks for Short and Long Term Recordings." In 2015 7th International IEEE/EMBS Conference on Neural Engineering (NER), 1000-1003. IEEE. doi: 10.1109/NER.2015.7146795.
- Ben-Ari, Yehezkel. 2001. "Developing Networks Play a Similar Melody." Trends in Neurosciences 24 (6). Elsevier: 353-60. doi:10.1016/S0166-2236(00)01813-0.
- Ben-Ari, Yehezkel. 2002. "Excitatory Actions of Gaba during Development: The Nature of the Nurture." Nature Reviews. Neuroscience 3 (September): 728-39. doi:10.1038/nrn920.
- Berdondini, L, P D van der Wal, O Guenat, N F de Rooij, M Koudelka-Hep, P Seitz, R Kaufmann, P Metzler, N Blanc, and S Rohr. 2005. "High-Density Electrode Array for Imaging in Vitro Electrophysiological Activity." Biosensors & Bioelectronics 21 (1): 167-74. doi:10.1016/j.bios.2004.08.011.
- Berdondini, L., T. Overstolz, N.F. de Rooij, M. Koudelka-Hep, M. Wany, and P. Seitz. 2001. "High-Density Microelectrode Arrays for Electrophysiological Activity Imaging of Neuronal Networks." In ICECS 2001. 8th IEEE International Conference on Electronics, Circuits and Systems (Cat. No.01EX483), 3:1239-42. IEEE. doi: 10.1109/ICECS.2001.957439.
- Berdondini, Luca, Kilian Imfeld, Alessandro Maccione, Mariateresa Tedesco, Simon Neukom, Milena Koudelka-Hep, and Sergio Martinoia. 2009. "Active Pixel Sensor Array for High Spatio-Temporal Resolution Electrophysiological Recordings from Single Cell to Large Scale Neuronal Networks." Lab on a Chip 9 (18). The Royal Society of Chemistry: 2644-51. doi:10.1039/b907394a.
- Buzsáki, György, and Kenji Mizuseki. 2014. "The Log-Dynamic Brain: How Skewed Distributions Affect Network Operations." Nature Reviews. Neuroscience 15 (4). Nature Publishing Group: 264-78. doi:10.1038/nrn3687.
- Chatzidaki, Anna, Antoine Fouillet, Jingling Li, Jeffrey Dage, Neil S Millar, Emanuele Sher, and Daniel Ursu. 2015. "Pharmacological Characterisation of Nicotinic Acetylcholine Receptors Expressed in Human iPSC-Derived Neurons." PloS One 10 (4): e0125116. doi:10.1371/journal.pone.0125116.
- Chiappalone, Michela, Marco Bove, Alessandro Vato, Mariateresa Tedesco, and Sergio Martinoia. 2006. "Dissociated Cortical Networks Show Spontaneously Correlated Activity Patterns during in Vitro Development." Brain Research 1093: 41-53. doi:10.1016/j.brainres.2006.03.049.
- Dage, Jeffrey L., Ellen M. Colvin, Antoine Fouillet, Emily Langron, William C. Roell, Jingling Li, Sachin X. Mathur, et al. 2014. "Pharmacological Characterisation of Ligand- and Voltage-Gated Ion Channels Expressed in Human iPSC-Derived Forebrain Neurons." Psychopharmacology 231 (2014): 1105-24. doi:10.1007/s00213-013-3384-2.
- Dayan, Peter, and L. F. Abbott. 2005. "Theoretical Neuroscience: Computational and Mathematical Modeling of Neural Systems," September. The MIT Press. http://dl.acm.org/citation.cfm?id=1205781.
   Dodla, Mahesh C, Jennifer Mumaw, and Steven L Stice. 2010. "Role of Astrocytes, Soluble Factors, Cells Adhesion Molecules and Neurotrophins in Functional Synapse Formation: Implications for Human Embryonic Stem Cell Derived Neurons." Current Stem Cell Research & Therapy 5 (3): 251-60. doi:10.2174/157488810791824520.
- Ehrlich, Ingrid, Matthew Klein, Simon Rumpel, and Roberto Malinow. 2007. "PSD-95 Is Required for Activity-Driven Synapse Stabilization." Proceedings of the National Academy of Sciences of the United States of America 104 (10): 4176-81. doi:10.1073/pnas.0609307104.
- El-Husseini, A E, E Schnell, D M Chetkovich, R A Nicoll, and D S Bredt. 2000. "PSD-95 Involvement in Maturation of Excitatory Synapses." Science (New York, N.Y.) 290 (5495): 1364-68. http://www.ncbi.nlm.nih.gov/pubmed/11082065.
- Espuny-Camacho, Ira, Kimmo A Michelsen, David Gall, Daniele Linaro, Anja Hasche, Jérôme Bonnefont, Camilia Bali, et al. 2013. "Pyramidal Neurons Derived from Human Pluripotent Stem Cells Integrate Efficiently into Mouse Brain Circuits in Vivo." Neuron 77 (3): 440-56. doi:10.1016/j.neuron.2012.12.011.
- Falcão, Ana S, Adelaide Fernandes, Maria A Brito, Rui F M Silva, and Dora Brites. 2006. "Bilirubin-Induced Immunostimulant Effects and Toxicity Vary with Neural Cell Type and Maturation State." Acta Neuropathologica 112 (1): 95-105. doi:10.1007/s00401-006-0078-4.
- Gerstein, George L., and Nelson Y.-S. Kiang. 1960. "An Approach to the Quantitative Analysis of Electrophysiological Data from Single Neurons." Biophysical Journal 1 (1). Elsevier: 15-28. doi:10.1016/S0006-3495(60)86872-5.
- Gill, JasKiran K, Anna Chatzidaki, Daniel Ursu, Emanuele Sher, and Neil S Millar. 2013. "Contrasting Properties of α7-Selective Orthosteric and Allosteric Agonists Examined on Native Nicotinic Acetylcholine Receptors." PloS One 8 (1): e55047. doi: 10.1371/journal.pone.0055047.
- Haythornthwaite, a., S. Stoelzle, a. Hasler, a. Kiss, J. Mosbacher, M. George, a. Bruggemann, and N. Fertig. 2012. "Characterizing Human Ion Channels in Induced Pluripotent Stem Cell-Derived Neurons." Journal of Biomolecular Screening 17 (X): 1264-72. doi:10.1177/1087057112457821.
- Hunt, C A, L J Schenker, and M B Kennedy. 1996. "PSD-95 Is Associated with the Postsynaptic Density and Not with the Presynaptic Membrane at Forebrain Synapses." The Journal of Neuroscience : The Official Journal of the Society for Neuroscience 16 (4): 1380-88. http://www.ncbi.nlm.nih.gov/pubmed/8778289.
- Ide, A N, A Andruska, M Boehler, B C Wheeler, and G J Brewer. 2010. "Chronic Network Stimulation Enhances Evoked Action Potentials." Journal of Neural Engineering 7 (1): 16008. doi:10.1088/1741-2560/7/1/016008.
- Jimbo, Yasuhiko, and Akio Kawana. 1992. "Electrical Stimulation and Recording from Cultured Neurons Using a Planar Electrode Array." Bioelectrochemistry and Bioenergetics 29 (2): 193-204. doi:10.1016/0302-4598(92)80067-Q.
- Jungblut, Melanie, Wolfgang Knoll, Christiane Thielemann, and Mark Pottek. 2009. "Triangular Neuronal Networks on Microelectrode Arrays: An Approach to Improve the Properties of Low-Density Networks for Extracellular Recording." Biomedical Microdevices 11 (6): 1269-78. doi:10.1007/s10544-009-9346-0.
- Lau, Pak-Ming, and Guo-Qiang Bi. 2005. "Synaptic Mechanisms of Persistent Reverberatory Activity in Neuronal Networks." Proceedings of the National Academy of Sciences of the United States of America 102 (29): 10333-38. doi:10.1073/pnas.0500717102.
- Levinson, Joshua N, and Alaa El-Husseini. 2005. "New Players Tip the Scales in the Balance between Excitatory and Inhibitory Synapses." Molecular Pain 1: 12. doi:10.1186/1744-8069-1-12.
- Llinas, R. 1988. "The Intrinsic Electrophysiological Properties of Mammalian Neurons: Insights into Central Nervous System Function." Science 242 (4886): 1654-64. doi:10.1126/science.3059497.
- Maccione, Alessandro, Mauro Gandolfo, Paolo Massobrio, Antonio Novellino, Sergio Martinoia, and Michela Chiappalone. 2009. "A Novel Algorithm for Precise Identification of Spikes in Extracellularly Recorded Neuronal Signals." Journal of Neuroscience Methods 177 (1): 241-49. doi:10.1016/j.jneumeth.2008.09.026.
- Maccione, Alessandro, Mauro Gandolfo, Mariateresa Tedesco, Thierry Nieus, Kilian Imfeld, Sergio Martinoia, and Luca Berdondini. 2010. "Experimental Investigation on Spontaneously Active Hippocampal Cultures Recorded by Means of High-Density MEAs: Analysis of the Spatial Resolution Effects." Frontiers in Neuroengineering 3 (May): 4. doi:10.3389/fneng.2010.00004.
- Maccione, Alessandro, Mauro Gandolfo, Stefano Zordan, Hayder Amin, Stefano Di Marco, Thierry Nieus, Gian Nicola Angotzi, and Luca Berdondini. 2015. "Microelectronics, Bioinformatics and Neurocomputation for Massive Neuronal Recordings in Brain Circuits with Large Scale Multielectrode Array Probes." Brain Research Bulletin 119 (Pt B): 118-26. doi:10.1016/j.brainresbull.2015.07.008.
- Matsuzawa, M, F F Weight, R S Potember, and P Liesi. 1996. "Directional Neurite Outgrowth and Axonal Differentiation of Embryonic Hippocampal Neurons Are Promoted by a Neurite Outgrowth Domain of the B2-Chain of Laminin." International Journal of Developmental Neuroscience: The Official Journal of the International Society for Developmental Neuroscience 14 (3): 283-95. http://www .ncbi.nlm.nih.gov/pubmed/8842805.
- Nam, Yoonkey, and Bruce C Wheeler. 2011. "In Vitro Microelectrode Array Technology and Neural Recordings." Critical Reviews in Biomedical Engineering 39 (1): 45-61. doi:10.1615/CritRevBiomedEng.v39.i1.40.
- Odawara, a., Y. Saitoh, a. H. Alhebshi, M. Gotoh, and I. Suzuki. 2014. "Long-Term Electrophysiological Activity and Pharmacological Response of a Human Induced Pluripotent Stem Cell-Derived Neuron and Astrocyte Co-Culture." Biochemical and Biophysical Research Communications 443 (4). Elsevier Inc.: 1176-81. doi:10.1016/j.bbrc.2013.12.142.
- Ohara, Yuki, Noriko Koganezawa, Hiroyuki Yamazaki, Reiko T. Roppongi, Kaoru Sato, Yuko Sekino, and Tomoaki Shirao. 2015. "Early-Stage Development of Human Induced Pluripotent Stem Cell-Derived Neurons." Journal of Neuroscience Research 93 (12): 1804-13. doi:10.1002/jnr.23666.
- Okabe, S, H D Kim, A Miwa, T Kuriu, and H Okado. 1999. "Continual Remodeling of Postsynaptic Density and Its Regulation by Synaptic Activity." Nature Neuroscience 2 (9): 804-11. doi:10.1038/12175.
- Orloff, John, Frank Douglas, Jose Pinheiro, Susan Levinson, Michael Branson, Pravin Chaturvedi, Ene Ette, et al. 2009. "The Future of Drug Development: Advancing Clinical Trial Design." Nature Reviews. Drug Discovery 8 (12). Nature Publishing Group: 949-57. doi:10.1038/nrd3025.
- Prange, Oliver, Tak Pan Wong, Kimberly Gerrow, Yu Tian Wang, and Alaa El-Husseini. 2004. "A Balance between Excitatory and Inhibitory Synapses Is Controlled by PSD-95 and Neuroligin." Proceedings of the National Academy of Sciences of the United States of America 101 (38): 13915-20. doi:10.1073/pnas.0405939101.
- Prodanov, Dimiter, Joost Heeroma, and Enrico Marani. 2006. "Automatic Morphometry of Synaptic Boutons of Cultured Cells Using Granulometric Analysis of Digital Images." Journal of Neuroscience Methods 151 (2): 168-77. doi:10.1016/j.jneumeth.2005.07.011.
- Rajamohan, Divya, Elena Matsa, Spandan Kalra, James Crutchley, Asha Patel, Vinoj George, and Chris Denning. 2013. "Current Status of Drug Screening and Disease Modelling in Human Pluripotent Stem Cells." BioEssays : News and Reviews in Molecular, Cellular and Developmental Biology 35: 281-98. doi:10.1002/bies.201200053.
- Sans, N, R S Petralia, Y X Wang, J Blahos, J W Hell, and R J Wenthold. 2000. "A Developmental Change in NMDA Receptor-Associated Proteins at Hippocampal Synapses." The Journal of Neuroscience: The Official Journal of the Society for Neuroscience 20 (3): 1260-71. http://www.ncbi.nlm.nih.gov/pubmed/10648730.
- Schneider, Caroline A, Wayne S Rasband, and Kevin W Eliceiri. 2012. "NIH Image to ImageJ: 25 Years of Image Analysis." Nature Methods 9 (7). Nature Publishing Group, a division of Macmillan Publishers Limited. All Rights Reserved.: 671-75. doi:10.1038/nmeth.2089.
- Shoham, Shy, Matthew R Fellows, and Richard A Normann. 2003. "Robust, Automatic Spike Sorting Using Mixtures of Multivariate T-Distributions." Journal of Neuroscience Methods 127 (2): 111-22. http://www.ncbi.nlm.nih.gov/pubmed/12906941.
- Sun, Qian, and Gina G Turrigiano. 2011. "PSD-95 and PSD-93 Play Critical but Distinct Roles in Synaptic Scaling up and Down." The Journal of Neuroscience : The Official Journal of the Society for Neuroscience 31 (18): 6800-6808. doi:10.1523/JNEUROSCI.5616-10.2011.
- Sun, Yi, Zhuo Huang, Wenwen Liu, Kaixuan Yang, Kang Sun, Shige Xing, Dong Wang, Wei Zhang, and Xingyu Jiang. 2012. "Surface Coating as a Key Parameter in Engineering Neuronal Network Structures in Vitro." Biointerphases 7 (1-4): 1-14. doi:10.1007/s13758-012-0029-7.
- Taft, Christine E, and Gina G Turrigiano. 2014. "PSD-95 Promotes the Stabilization of Young Synaptic Contacts." Philosophical Transactions of the Royal Society of London. Series B, Biological Sciences 369 (1633): 20130134. doi:10.1098/rstb.2013.0134.
- Takahashi, Kazutoshi, Koji Tanabe, Mari Ohnuki, Megumi Narita, Tomoko Ichisaka, Kiichiro Tomoda, and Shinya Yamanaka. 2007. "Induction of Pluripotent Stem Cells from Adult Human Fibroblasts by Defined Factors." Cell 131 (5): 861-72. doi:10.1016/j.cell.2007.11.019.
- Teramae, By Jun-nosuke, and Tomoki Fukai. 2014. "Computational Implications of Lognormally Distributed Synaptic Weights," no. April.
- Turrigiano, Gina G, and Sacha B Nelson. 2004. "Homeostatic Plasticity in the Developing Nervous System." Nature Reviews. Neuroscience 5: 97-107. doi:10.1038/nrn1327.
- van den Bogaart, Geert, Karsten Meyenberg, H. Jelger Risselada, Hayder Amin, Katrin I. Willig, Barbara E. Hubrich, Markus Dier, et al. 2011. "Membrane Protein Sequestering by Ionic Protein-lipid Interactions." Nature 479 (7374): 552-55. doi:10.1038/nature10545.
- Wagenaar, Daniel A, Jerome Pine, and Steve M Potter. 2004. "Effective Parameters for Stimulation of Dissociated Cultures Using Multi-Electrode Arrays." Journal of Neuroscience Methods 138 (1-2): 27-37. doi:10.1016/j.jneumeth.2004.03.005.
- Wagenaar, Daniel a, Jerome Pine, and Steve M Potter. 2006. "An Extremely Rich Repertoire of Bursting Patterns during the Development of Cortical Cultures." BMC Neuroscience 7: 11. doi:10.1186/1471-2202-7-11.
- Wheeler, B. C., J. M. Corey, G. J. Brewer, and D. W. Branch. 1999. "Microcontact Printing for Precise Control of Nerve Cell Growth in Culture." Journal of Biomechanical Engineering 121 (1). American Society of Mechanical Engineers: 73. doi:10.1115/1.2798045.
- Whitemarsh, R. C. M., M. J. Strathman, L. G. Chase, C. Stankewicz, W. H. Tepp, E. A. Johnson, and S. Pellett. 2012. "Novel Application of Human Neurons Derived from Induced Pluripotent Stem Cells for Highly Sensitive Botulinum Neurotoxin Detection." Toxicological Sciences 126 (2): 426-35. doi:10.1093/toxsci/kfr354.
- Wonders, Carl P, and Stewart A Anderson. 2006. "The Origin and Specification of Cortical Interneurons." Nature Reviews. Neuroscience 7 (9): 687-96. doi:10.1038/nrn1954.
- Yassin, Lina, Brett L. Benedetti, Jean-Sebastien Jouhanneau, Jing a. Wen, James F.a. Poulet, and Alison L. Barth. 2010. "An Embedded Subnetwork of Highly Active Neurons in the Neocortex." Neuron 68 (6): 1043-50. doi:10.1016/j.neuron.2010.11.029.
- Ziff, Edward B. 1997. "Enlightening the Postsynaptic Density." Neuron 19 (6): 1163-74. doi:10.1016/S0896-6273(00)80409-2.

## Claims

1. A neuronal cell culture substrate, **characterized in that** it comprises a neuronal cell culture support surface coated with poly-DL-ornithine alone, wherein said poly-DL-ornithine has a molecular weight of 30,000 or higher.

2. A neuronal cell culture substrate according to claim 1, wherein said neuronal cell is a primary neuron or an iPSC-derived neuronal cell line.

3. A neuronal cell culture substrate according to claim 2, wherein said neuronal cell is a primary mammalian neuron.

4. The neuronal cell culture substrate according to any of claims 1 to 3, wherein said neuronal cell culture support surface coated with poly-DL-ornithine is made of glass, silicon, polystyrene or metal.

5. An article comprising a neuronal cell culture substrate according to any of claims 1 to 4.

6. The article according to claim 5, which is a neuronal cell culture vessel, a bio-sensing device or a multi-electrode or micro-electrode array, preferably a planar micro-electrode array.

7. The article according to claim 6, which is a neuronal cell culture vessel selected from the group consisting of a slide, a plate, a flask, a bottle and a bioreactor.

8. The article according to claim 6, which is a complementary-metal-oxide semiconductor (CMOS)-multi-electrode array or bio-sensing device.

9. An *in vitro* method of promoting adhesion, survival and/or proliferation of neuronal cells in culture, the method comprising culturing neuronal cells on a neuronal cell culture substrate according to any of claims 1 to 4 or on/in an article according to any of claims 5 to 8.

10. An *in vitro* method of promoting neuronal network growth and/or maturation, the method comprising culturing neuronal cells on a neuronal cell culture substrate according to any of claims 1 to 4 or on/in an article according to any of claims 5 to 8.

11. The *in vitro* method according to claim 9 or 10, wherein said neuronal cells are primary neurons or a iPSC-derived neuronal cell line.

12. The in vitro method according to any of claims 9 to 11, wherein said neuronal cells are primary mammalian neurons.

13. The *in vitro* method according to any of claims 9 to 12, wherein said neuronal cells are rat, mouse or human neurons.

## Patentansprüche

1. Kultursubstrat für neuronale Zellen, **dadurch gekennzeichnet, dass** es eine Trägeroberfläche für die Kultur von neuronalen Zellen umfasst, die nur mit Poly-DL-Ornithin beschichtet ist, wobei das Poly-DL-Ornithin ein Molekulargewicht von 30.000 oder höher aufweist.

2. Kultursubstrat für neuronale Zellen nach Anspruch 1, wobei die neuronale Zelle ein primäres Neuron oder eine von iPSCs abgeleitete neuronale Zelllinie ist.

3. Kultursubstrat für neuronale Zellen nach Anspruch 2, wobei die neuronale Zelle ein primäres Neuron eines Säugetiers ist.

4. Kultursubstrat für neuronale Zellen nach einem der Ansprüche 1 bis 3, wobei die mit Poly-DL-Ornithin beschichtete Trägeroberfläche für die Kultur von neuronalen Zellen aus Glas, Silizium, Polystyrol oder Metall besteht.

5. Gegenstand, umfassend ein Kultursubstrat für neuronale Zellen nach einem der Ansprüche 1 bis 4.

6. Gegenstand nach Anspruch 5, der ein Gefäß für die Kultur von neuronalen Zellen, eine Biosensor-Vorrichtung oder ein Multi-Elektroden- oder Mikro-Elektroden-Array, vorzugsweise ein planares Mikro-Elektroden-Array, ist.

7. Gegenstand nach Anspruch 6, der ein Gefäß für die Kultur von neuronalen Zellen ist, ausgewählt aus der Gruppe, bestehend aus einem Objektträger, einer Platte, einem Kolben, einer Flasche und einem Bioreaktor.

8. Gegenstand nach Anspruch 6, der ein komplementärer Metalloxid-Halbleiter (CMOS)-Multi-Elektroden-Array oder eine Biosensor-Vorrichtung ist.

9. *In vitro*-Verfahren zur Förderung der Adhäsion, des Überlebens und/oder der Proliferation von neuronalen Zellen in Kultur, wobei das Verfahren die Kultivierung von neuronalen Zellen auf einem Kultursubstrat für neuronale Zellen nach einem der Ansprüche 1 bis 4 oder auf/in einem Gegenstand nach einem der Ansprüche 5 bis 8 umfasst.

10. *In vitro*-Verfahren zur Förderung des Wachstums und/oder der Reifung von neuronalen Netzwerken, wobei das Verfahren die Kultivierung von neuronalen Zellen auf einem Kultursubstrat für neuronale Zellen nach einem der Ansprüche 1 bis 4 oder auf/in einem Gegenstand nach einem der Ansprüche 5 bis 8 umfasst.

11. *In vitro*-Verfahren nach Anspruch 9 oder 10, wobei die neuronalen Zellen primäre Neuronen oder eine von iPSCs abgeleitete neuronale Zelllinie sind.

12. *In vitro*-Verfahren nach einem der Ansprüche 9 bis 11, wobei die neuronalen Zellen primäre Neuronen eines Säugetiers sind.

13. *In vitro*-Verfahren nach einem der Ansprüche 9 bis 12, wobei die neuronalen Zellen Ratten-, Maus- oder menschliche Neuronen sind.

## Revendications

1. Substrat de culture de cellules neuronales, **caractérisé en ce qu'**il comprend une surface de support de culture de cellules neuronales revêtue d'une poly-DL-ornithine seule, dans lequel ladite poly-DL-ornithine présente un poids moléculaire de 30 000 ou plus.

2. Substrat de culture de cellules neuronales selon la revendication 1, dans lequel ladite cellule neuronale est un neurone primaire ou une lignée de cellules neuronales dérivées d'iPSC.

3. Substrat de culture de cellules neuronales selon la revendication 2, dans lequel ladite cellule neuronale est un neurone primaire de mammifère.

4. Substrat de culture de cellules neuronales selon l'une quelconque des revendications 1 à 3, dans lequel ladite surface de support de culture de cellules neuronales revêtue d'une poly-DL-ornithine est faite de verre, de silicium, de polystyrène ou de métal.

5. Article comprenant un substrat de culture de cellules neuronales selon l'une quelconque des revendications 1 à 4.

6. Article selon la revendication 5, qui est un récipient de culture de cellules neuronales, un dispositif de biodétection ou une matrice de multi-électrodes ou de micro-électrodes, de préférence une matrice plane de micro-électrodes.

7. Article selon la revendication 6, qui est un récipient de culture de cellules neuronales sélectionné dans le groupe constitué d'une lame, d'une plaque, d'un flacon, d'une bouteille et d'un bioréacteur.

8. Article selon la revendication 6, qui est une matrice de multi-électrodes ou un dispositif de biodétection à semi-conducteur à oxyde de métal complémentaire (CMOS).

9. Procédé *in vitro* pour favoriser l'adhérence, la survie et/ou la prolifération de cellules neuronales en culture, le procédé comprenant la mise en culture de cellules neuronales sur un substrat de culture de cellules neuronales selon l'une quelconque des revendications 1 à 4 ou sur/dans un article selon l'une quelconque des revendications 5 à 8.

10. Procédé *in vitro* pour favoriser la croissance et/ou la maturation d'un réseau neuronal, le procédé comprenant la mise en culture de cellules neuronales sur un substrat de culture de cellules neuronales selon l'une quelconque des revendications 1 à 4 ou sur/dans un article selon l'une quelconque des revendications 5 à 8.

11. Procédé *in vitro* selon la revendication 9 ou 10, dans lequel lesdites cellules neuronales sont des neurones primaires ou une lignée de cellules neuronales dérivées d'iPSC.

12. Procédé *in vitro* selon l'une quelconque des revendications 9 à 11, dans lequel lesdites cellules neuronales sont des neurones primaires de mammifère.

13. Procédé *in vitro* selon l'une quelconque des revendications 9 à 12, dans lequel lesdites cellules neuronales sont des neurones de rat, de souris ou d'humain.
